(19) Europäisches Patentamt
European Patent Office
Office européen des brevets

(11) EP 4 737 453 A1

(12) **EUROPEAN PATENT APPLICATION**
published in accordance with Art. 153(4) EPC

(43) Date of publication:
**06.05.2026 Bulletin 2026/19**

(21) Application number: **24832524.3**

(22) Date of filing: **28.06.2024**

(51) International Patent Classification (IPC):
***C07D 421/12*** (2006.01) ***A61K 31/517*** (2006.01)
***A61K 31/519*** (2006.01) ***A61P 35/00*** (2006.01)
***C07D 421/14*** (2006.01) ***C07D 487/04*** (2006.01)
***A61K 31/095*** (2006.01) ***A61K 31/5377*** (2006.01)
***A61K 31/675*** (2006.01) ***A61K 31/695*** (2006.01)

(52) Cooperative Patent Classification (CPC):
**A61K 31/095; A61K 31/517; A61K 31/519; A61K 31/5377; A61K 31/675; A61K 31/695; A61P 35/00; C07D 421/12; C07D 421/14; C07D 487/04**

(86) International application number:
**PCT/KR2024/009146**

(87) International publication number:
**WO 2025/005748 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.06.2023 KR 20230084377**
**17.06.2024 KR 20240078041**

(71) Applicant: **Aigen Sciences Inc.**
**Seoul 02841 (KR)**

(72) Inventors:
- **LEE, Kwang-Ok**
  **Seoul 04778 (KR)**
- **LEE, Ho-Jeong**
  **Seoul 04778 (KR)**
- **KIM, Sunkyu**
  **Seoul 04778 (KR)**
- **KIM, Jihye**
  **Seoul 04778 (KR)**
- **PARK, Sejeong**
  **Seoul 04778 (KR)**
- **LEE, Sanghoon**
  **Seoul 04778 (KR)**
- **YOO, Kiwoong**
  **Seoul 04778 (KR)**
- **YOO, Hyerim**
  **Seoul 04778 (KR)**

(74) Representative: **Germain Maureau**
**12, rue Boileau**
**69006 Lyon (FR)**

(54) **NOVEL SELENOPHENE DERIVATIVES**

(57) Provided are a compound defined by chemical formula 1 or a tautomer thereof and pharmaceutically acceptable salts thereof. The compound of the present invention can inhibit SOS1 activity.



Processed by Luminess, 75001 PARIS (FR)

**(Cont. next page)**

【Chemical Formula 1】

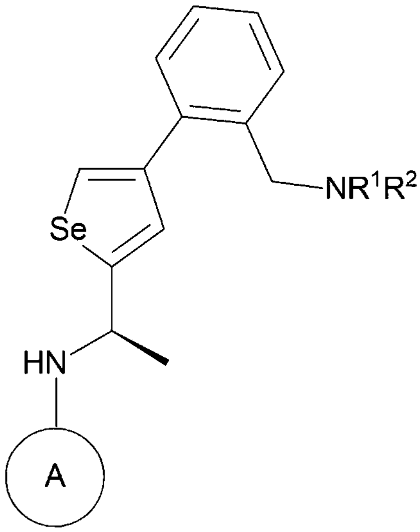

【FIG. 1】

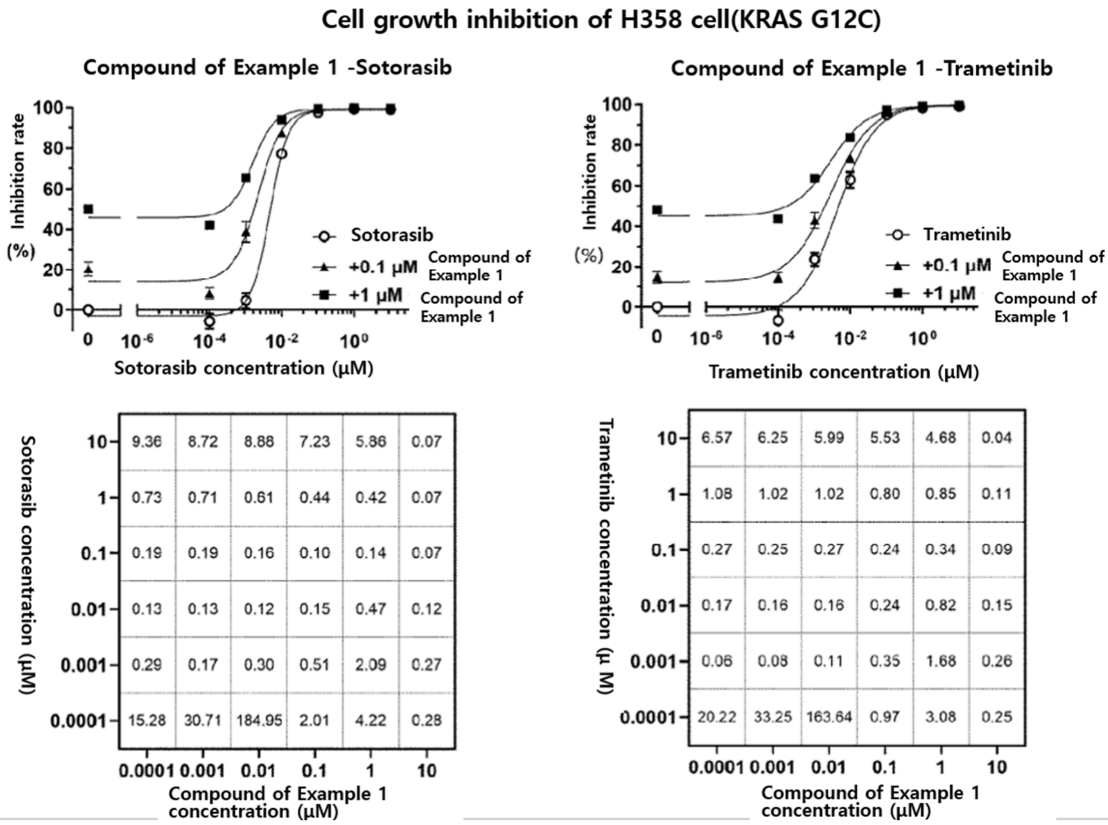

## Description

[TECHNICAL FIELD]

The present invention relates to small organic compounds having inhibitory activity against biological enzymes. The present invention also relates to a pharmaceutical composition containing such small organic compounds.

[BACKGROUND ART]

SOS1 (Son of Sevenless 1) is known as a key factor in the activation of small GTPases such as RAS (Rat Sarcoma viral oncogene homolog) and Rac1 (Ras-related C3 botulinum toxin substrate 1). These small GTPases are critical components of cell signaling pathways that regulate various cellular processes, including cell growth, differentiation, and migration.

SOS1 acts as a guanine nucleotide exchange factor (GEF) that promotes the exchange of GDP (guanosine diphosphate)-bound Ras to GTP (guanosine triphosphate)-bound Ras, thereby activating Ras. Activated Ras bound to GTP subsequently interacts with downstream effector proteins to initiate signaling pathways. The activation of Ras by SOS1 is a critical step in various cellular processes, such as cell proliferation, survival, and differentiation. Activated Ras then initiates downstream signaling pathways, such as the MAPK (Mitogen-Activated Protein Kinase) cascade, and thus regulating gene expression and cellular responses to external signals. Abnormal regulation of the Ras pathway is closely associated with mutations in tumors, making it a significant focus in cancer research.

RAS are subtyped to three isoforms of KRAS, NRAS, and HRAS. It is known that KRAS is the most frequently mutated among them, and that SOS1 plays a critical role in the activation of oncogenic signaling by mutant KRAS (Jeng et al., Nat. Commun., 2012, 3:1168). Specifically, depletion of intracellular SOS1 concentration led to decreased proliferation rate and viability in tumor cells having KRAS mutations, whereas no such effect was observed in cell lines with wild-type KRAS. Moreover, the detrimental effects resulting from deletion of SOS1 could not be rescued by introducing SOS1 having a modified catalytic region, demonstrating that the GEF activity of SOS1 is essential for the survival of KRAS-mutant cancer cells.

Besides RAS, SOS1 is also involved in the activation of Rac1. Rac1 is another small GTPase that belongs to the Rho protein family and plays a crucial role in regulating the cytoskeleton, cell migration, and other cellular processes. SOS1 acts by exchanging GDP-bound on Rac1 for GTP, thereby switching it to its active state. Activated Rac1 involves in processes such as cell adhesion, migration, and invasion, and is known to play a significant role in both normal cellular functions and pathological conditions such as cancer metastasis.

In view of these points, SOS1 inhibitors may be developed as therapeutic drugs for diseases caused by KRAS mutations or Rac1 activation.

[PRIOR ART DOCUMENT]

NON-PATENT DOCUMENT: Jeng et al., Nat. Commun., 2012, 3:1 168

[DISCLOSURE]

[TECHNICAL PROBLEM]

An object of the present invention is to provide a novel selenophene derivative compound that is an SOS1 inhibitor with excellent activity in inhibiting protein-protein interaction of KRAS-SOS1. Another object of the present invention is to provide a pharmaceutical composition comprising the compound in a therapeutically effective amount.

[TECHNICAL SOLUTION]

In one aspect of the present invention, a selenophene compound defined by chemical formula 1 having an inhibitory activity on SOS1 or a tautomer thereof and pharmaceutically acceptable salts thereof are provided.

【Chemical Formula 1】

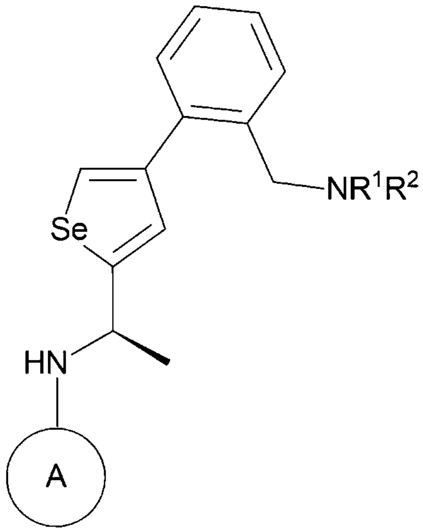

In the Chemical Formula 1, $R^1$ and $R^2$ are functional groups independently selected from the group consisting of H, $C_{1\sim4}$ alkyl, and $C_{1\sim4}$ alkyl substituted with deuterium, or $R^1$ and $R^2$ form a 4 to 6 membered heterocyclic alkyl ring together with nitrogen atom to which $R^1$ and $R^2$ are bonded, except that both of $R^1$ and $R^2$ are H,

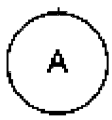

is

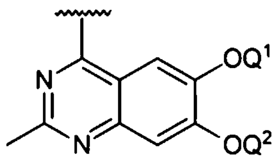 , 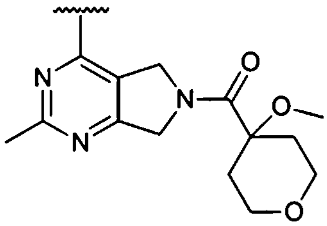 or 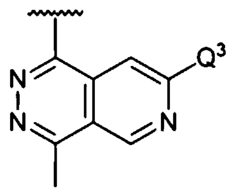

**wherein** $Q^1$ and $Q^2$ are functional groups independently selected from $C_{1\sim4}$ alkyl and 4 to 8 membered monocyclic heterocyclyl, $Q^3$ is 4 to 8 membered monocyclic heterocyclyl, and symbol "



" indicates that Ⓐ is connected to the remaining part of the compound at the position.

In another aspect of the present invention, there is provided a pharmaceutical composition for treatment of cancer comprising a therapeutically effective amount of the compound described above, a tautomer thereof, or a pharmaceutically acceptable salt thereof, and a pharmaceutically acceptable excipient.

In another aspect of the present invention, a method for treating cancer is provided. The method comprises administering to a patient a therapeutically effective amount of a compound of the present invention, such as a compound defined by Chemical Formula 1, or a pharmaceutical composition comprising such a compound of the present invention.

[ADVANTAGOUS EFFECT]

The compound of the present invention can inhibit an activity of SOS1. Furthermore, the compound of the present invention exhibits a synergistic cell death effect in conjunction with blockade of RAS signaling or EGFR signaling. In a

preferred embodiment, the compound of the present invention exhibits selective inhibitory activity against SOS1 over SOS2. The compound of the present invention is useful for cancer treatment.

[BRIEF DESCRIPTION OF DRAWINGS]

Fig. 1 is a dose-response curve for cell growth inhibition showing the synergistic effect observed when the compound of the present invention has been used in combination with a targeted anticancer agent blocking RAS signaling in the NCI-H358 cancer cell line.

Fig. 2 is a dose-response curve for cell growth inhibition showing the synergistic effect observed when the compound of the present invention has been used in combination with a targeted anticancer agent blocking EGFR signaling in the PC-9 cancer cell line.

Fig. 3 is a graph showing that the compound of the present invention increases the production of reactive oxygen species in MIA-PaCa-2 cancer cells.

[MODE FOR INVENTION]

Hereinafter, embodiments of the present invention will be described in detail. Prior to this, the terms and words used in this specification and claims should not be construed as limited to their conventional or dictionary meanings. Based on the principle that inventors can appropriately define the concepts of terms to best explain their inventions, they should be interpreted in a way that aligns with the technical concept of the present invention.

Therefore, the embodiments described in this specification are merely examples presented to aid understanding of the present invention and do not fully represent the technical concept of the present invention. Therefore, it should be understood that various equivalents and modifications may exist at the time of filing.

Unless otherwise defined, all technical terms used in this specification have the same meaning as commonly understood by those skilled in the art. While preferred methods and samples are described herein, similar or equivalent methods are also included within the scope of the present invention. Numerical values described herein are assumed to include the meaning of "about," even if not explicitly stated. The numerical range indicated using the term "to" in this specification includes the ranges that include the numerical values described before and after the term "to" as the lower and upper limits, respectively.

Definition of term

In the present specification, the term "suppression of SOS1" or "inhibition of SOS1" or "suppression of SOS1 activity" or "inhibition of SOS1 activity" or the term "SOS1 suppressor" or "SOS1 inhibitor" for the compounds refers to action of reducing the guanine nucleotide exchange activity of SOS1 for RAS or by blocking the binding of RAS to SOS1. For example, "SOS1 suppressor" or "SOS1 inhibitor" is a substance that has an action of reducing the measured signal of guanine nucleotide exchange activity of SOS1 or measured signal of binding of SOS1 to RAS by at least about 10%, at least about 20%, at least about 30%, at least about 40%, at least about 50%, at least about 60%, at least about 70%, at least about 80%, at least about 90%, or at least about 95%, when measured under comparable conditions with a negative control (e.g., a blank).

Those skilled in the art will recognize that some of the compounds of the present invention may exist in more than one tautomeric form. Since a single chemical structural formula can only represent a single tautomeric form, it is well understood that when a compound is referred to by a single structural formula for convenience, the structural formula encompasses all tautomeric variations of the compound. Depending on the compound, one tautomer may be present primarily, a mixture of multiple tautomers may exist at room temperature, or a single tautomer may be isolated. Examples of tautomers include those between the pyridone and hydroxypyridine forms, and those between the keto and enol forms.

In this specification, the prefix of "$C_{x-y}$" or "$C_x$-$C_y$" (where x and y are natural numbers) is used before a functional group to indicate the number of carbon atoms that the functional group has in its backbone. For example, a $C_{1-4}$ alkyl group indicates an alkyl group having 1 to 4 carbon atoms, and a $C_{1-3}$ alkyl group indicates an alkyl group having 1 to 3 carbon atoms, respectively. For example, C1-C4 alkoxy groups include methoxy, ethoxy, n-propoxy, i-propoxy, n-butoxy, sec-butoxy, and t-butoxy. For example, a $C_3$-$C_6$ cycloalkyl group includes cyclopropyl, cyclobutyl, cyclopentyl, or cyclohexyl groups. In addition, in the present specification, when the number of elements forming a ring or the number of rings is indicated in front of a cyclic functional group or cyclic compound (including both carbon rings and heterocycles), the ring is a ring formed of the corresponding number of elements, i.e., carbon and/or heteroatoms, or a structure having the corresponding number of rings. For example, a six-membered cyclic heteroaryl refers to a heteroaryl group that contains one or more heteroatoms which forms a ring with carbon, and has six elements of the ring. Also, for example, a 4 to 6

membered monocyclic heteroaryl refers to a monocyclic aromatic ring that is formed of only one ring rather than a fused ring, bridged ring, or spirocyclic compound, and the sum of the number of carbons and heteroatoms forming the ring backbone is 4 to 6, such as pyrrolyl, pyridinyl, imidazolyl, isoxazolyl, thiophenyl, thiazolyl, and the like.

Unless otherwise specified herein, when a functional group is linked to another part of a compound by a bond, the linkage may be via any atom of the functional group, provided that it is a suitable atom. For example, when referring to a propyl group, both prop-1-yl and prop-2-yl are included.

The term "halogen" in this specification refers to an atom selected from fluorine, chlorine, bromine, and iodine.

The term "$C_{1-6}$ alkyl" in this specification refers to a monovalent saturated hydrocarbon group with the chemical formula of $C_nH_{2n+1}$, where n is a natural number from 1 to 6. $C_{1-6}$ alkyl group encompasses any linear or branched saturated hydrocarbon group containing 1 to 6 carbon atoms, such as n-propyl, i-propyl, 2-methyl-ethyl, sec-butyl, tert-butyl, and the like.

As used herein, the term "$C_{1-6}$ alkylene" refers to a divalent saturated hydrocarbon group with the chemical formula of CnH2n, where n is a natural number of 1 to 6.

As used herein, the term "cycloalkyl" refers to a monovalent saturated hydrocarbon radical containing one or more carbon rings. A monocyclic cycloalkyl group containing only one carbon ring has the chemical formula of $C_nH_{2n-1}$. Examples of monocyclic $C_{3-6}$ cycloalkyl groups include cyclopropyl, cyclobutyl, cyclopentyl, and cyclohexyl.

In this specification, the term "bicycle" refers to a carbocyclic compound or heterocyclic compound composed of two rings, which are formed by sharing one or more atoms of rings between the two constituent rings. In a bicyclic compound, the atom shared by the two rings is called a bridgehead atom. Bicyclic compounds are divided into three types depending on the number of bridgehead atoms and whether or not there is a direct connection between the bridgehead atoms. A fused bicyclic compound has two bridgehead atoms that are directly connected to each other by a bond. Examples of fused bicyclic compounds include decalin, naphthalene, anthracene, phenanthrene, indole, benzofuran, purine, and quinoline. A cyclic compound with only one bridgehead atom is a spirocyclic compound. A bridged ring compound has two bridgehead atoms that are not directly connected to each other by a bond, but rather have one or more atoms of rings interposed between the two bridgehead atoms. Examples of bridged cyclic compounds include norbornane, 7-oxabicyclo[2.2.1]heptane, and adamantane.

In this specification, fused bicyclic compounds can be expressed by specifying the number of atoms in each of the two rings that constitute the fused bicyclic compound. For example, thienopyridine, benzofuran, indole, etc. can be referred to as five- or six-membered fused bicyclic compounds. Similarly, naphthalene, chromane, tetrahydroquinoline, quinoline, quinoxaline, pteridine, etc. can be referred to as six-membered and six-membered fused bicyclic compounds.

The term "carbocyclyl" as used herein refers to a hydrocarbon group having a saturated or unsaturated ring that is not aromatic, has a ring size of 3 to 14 members, and a monovalent radical in which the ring backbone is composed of only carbon atoms without any heteroatoms. As used herein, saturated carbocyclyl includes not only a monocyclic functional group but also a polycyclic saturated carbocyclyl, for example, a bicyclic hydrocarbon group of a fused ring (fused), bridged ring (bridged), and spiro ring (spiro) saturated hydrocarbon ring. For example, some examples of exemplary saturated $C_{3-10}$ carbocyclyl groups include the aforementioned cycloheptyl ($C_7$), cyclooctyl ($C_8$), bicyclo[1.1.1]pentanyl ($C_5$), bicyclo[2.2.1]heptanyl (C7), bicyclo[2.2.2]octanyl (C8), cyclononyl (C9), cyclodecyl (C10), decahydronaphthalenyl (decalinyl) (C10), adamantyl (C10), spiro[4.5]decanyl (C10), etc. Unsaturated carbocyclyl refers to a hydrocarbon ring having one or more double bonds or triple bonds in the ring and is not aromatic. Examples of unsaturated carbocyclyl groups include cyclopentadienyl, cyclohexenyl, norbornenyl, and the like.

The term "aryl" as used herein refers to a monovalent hydrocarbon functional group that has an aromatic hydrocarbon ring of 6 to 14 carbon atoms in the ring backbone and contains one to three fused rings. Examples of aryl groups include phenyl, naphthyl, anthracenyl, and phenanthrenyl.

The term "heteroaryl" as used herein refers to a monovalent 5 to 12-membered unsaturated ring and an aromatic ring, which includes carbon atoms together with one or more heteroatoms selected from nitrogen, oxygen, and sulfur in the ring backbone. The ring system of the heteroaryl may be a single ring, or a double ring or triple ring in the form of a fused ring. The rings containing direct bonds between oxygen and sulfur atoms among the ring-constituting atoms, such as -O-O-, -O-S-, or -S-S-, are excluded from the term of heteroaryl. Some examples of heteroaryl compounds include pyrrolyl, imidazolyl, pyrazolyl, 2-pyridyl, 3-pyridyl, 4-pyridyl, pyrimidyl, triazolyl, quinazolinyl, 2-furanyl, 3-furanyl, benzofuranyl, 2-thienyl, oxazolyl, isothiazolyl, and thiadiazolyl. Heteroaryls can also be defined by their heteroatoms; for example, a heteroaryl in which the heteroatom is nitrogen would include pyrrolyl, imidazolyl, pyrazolyl, and 2-pyridyl, but would exclude furanyl and thienyl.

Fused bicyclic heteroaryl groups include six-membered and five-membered fused bicyclic heteroaryl groups such as benzimidazolyl, benzofuranyl, benzothiophenyl, isoindolyl, indazolyl, imidazopyridineyl, imidazopyrimidineyl, imidazopyridazinyl, indazolyl, pyrazoleopyridineyl, pyrrolopyrimidineyl, pyrrolopyridineyl, pyrrolopyrazinyl, triazolopyridineyl, triazolopyrimidineyl, purinyl, benzothiazolyl, benzoisothiazolyl, benzothiadiazolyl, benzoxazolyl, benzothiadiazolyl, etc., and six-membered and six-membered fused bicyclic heteroaryl compounds such as benzopyridineyl, benzopyrimidineyl, quinolinyl, isoquinolinyl, phthalazinyl, Examples include quinazolinyl, quinoxalinyl, naphthyridinyl,

quinolinzinyl, and cinnolinyl.

As used herein, the term "heteroarylene" refers to a divalent aromatic ring derived from the aforementioned heteroaryl group, by removing one hydrogen atom.

As used herein, the term "heterocycloalkyl" refers to a non-aromatic ring of a monovalent radical in which at least one carbon atom forming the cycloalkyl ring is independently replaced with a heteroatom selected from nitrogen, oxygen, and sulfur. Examples of heterocycloalkyl groups include pyrrolidinyl, imidazolidinyl, piperidinyl, pyrrolinyl, piperazinyl, morpholinyl, and thiazolidinyl.

The term "monocyclic heterocyclyl" as used herein refers to a monovalent unsaturated or saturated monocyclic ring, and radicals including at least one heteroatom selected from nitrogen, oxygen, and sulfur among the atoms.in ring backbone Therefore, monocyclic heterocyclyl also includes monocyclic heteroaryl, monocyclic heterocycloalkyl, and monocyclic heterocycles that include unsaturated bonds and heteroatoms in the ring backbone but are not aromatic.

In this specification, the term "substitution" or "substituted" in front of a functional group, unless otherwise stated in the specification, refers to a structure in which one or more hydrogens of the functional group are replaced by a designated functional group other than the hydrogen, assuming that the substituted functional group maintains its normal valence and produces a chemically-stable functional group. In typical cases, the substituent is selected from halogen, CN, OH, $C_{1-6}$ alkyl, $C_{3-6}$ cycloalkyl, $C_{3-14}$ heteroaryl, $C_{1-6}$ alkoxy and $CF_3$.

In this specification, when a specific atom of a substituent constituting a part of a compound is marked with symbol of "



", it indicates that the substituent is chemically bonded to the remaining part of the compound through that atom.

In this specification, when a bond line is marked with a dotted line such as "---" and, it indicates that the bond line is an optional element that may or may not be present. For example, the structure of

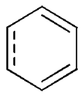

indicates that this structural formula includes benzene and 1,3-cyclohexadiene.

As used herein, the term "pharmaceutically acceptable salt" for a compound refers to a salt that does not impair the desired biological activity of the compound and does not cause inappropriate toxicity, irritation, or allergic reactions when used in contact with human or animal tissues. Pharmaceutically acceptable salts are well known in the art. For example, reference may be made to prior literature such as the paper by Berge et al., J. Pharmaceutical Sciences (1977), Vol. 66, pp. 1-19. Some examples of pharmaceutically acceptable salts include acid addition salts such as hydrochloride, hydrobromide, phosphate, sulfate, perchlorate, acetate, oxalate, maleate, tartrate, citrate, succinate, malonate, adipate, alginate, ascorbate, aspartate, benzoate, benzenesulfonate, and hydrosulfide, and base addition salts such as alkali metal salts, alkaline earth metal salts, ammonium salts, and quaternary ammonium ($N^+(C_{1-4}$ alkyl$)_4$). Examples of alkali metal or alkaline earth metal salts include sodium, lithium, potassium, calcium, and magnesium.

Compound

In one aspect of the present invention, there is provided a compound defined by Chemical Formula 1, a tautomer thereof, and pharmaceutically acceptable salts thereof.

【Chemical Formula 1】

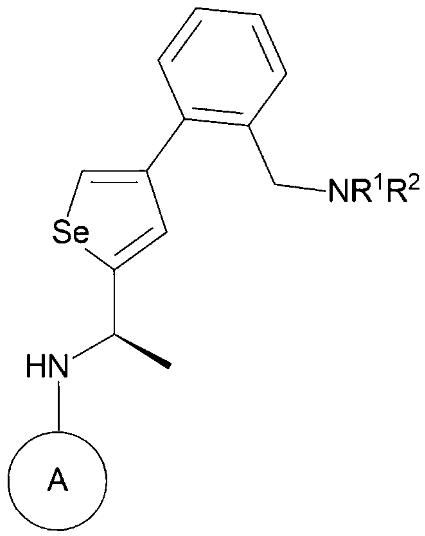

in the Chemical Formula 1, $R^1$ and $R^2$ are functional group independent selected from the group consisting of H, $C_{1-4}$ alky, and $C_{1-4}$ alkyl substituted with deuterium, or $R^1$ and $R^2$ form a 4 to 6 membered heterocyclic alkyl ring together with nitrogen atom to which $R^1$ and $R^2$ are bonded, except that both of $R^1$ and $R^2$ are H,

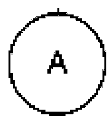

is

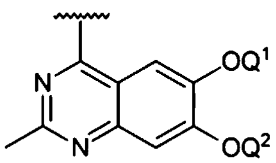

,

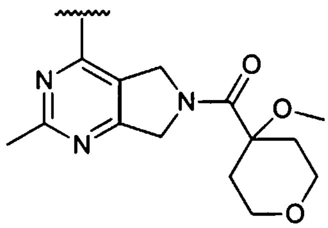

or

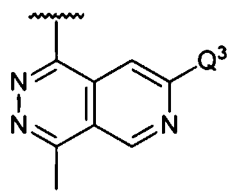

wherein $Q^1$ and $Q^2$ are functional groups independently selected from $C_{1\sim4}$ alkyl and 4 to 8 membered monocyclic heterocyclyl, $Q^3$ is 4 to 8 membered monocyclic heterocyclyl, and symbol "



" indicates that Ⓐ is connected to the corresponding position in the remaining part of the compound.

In an embodiment of the present invention, one of the $R^1$ and $R^2$ in the compound of Chemical Formula 1 is hydrogen, and the other is a methyl group or a trideuteriomethyl (CD3) group.

In another embodiment of the present invention, the 4-6 membered heterocyclo alkyl ring in the compound of Chemical Formula 1 is azetidinyl or pyrrolidinyl.

In another embodiment of the present invention, both Q1 and Q2 in the compound of Chemical Formula 1 are methyl, or one of Q1 and Q2 is methyl and the other is tetrahydrofuranyl.

In another embodiment of the present invention, all Q3 in the compound of Chemical Formula 1 are morpholinyl. In a more specific embodiment, the morpholinyl is N-morpholinyl.

In a more specific embodiment, the compounds of the present invention are the following compounds, stereoisomers, tautomers, solvates or pharmaceutically acceptable salts thereof:

(2-(2-cyclopropyl-6-methoxyphenyl)-4-((4-(1-methyl-4-(trifluoromethyl)-1H-imidazol-2-yl)benzyl)amino)pyrimidine-5-yl) dimethylphosphine oxide;

(4'-cyclopropyl-6'-methoxy-4-((4-(1-methyl-4-(trifluoromethyl)-1*H*-imidazol-2-yl)benzyl)amino)-[2,5'-bipyrimidine]-5-yl)dimethylphosphine oxide;
(2-(1-isopropyl-4-methyl-1*H*-pyrazole-5-yl)-4-((4-(1-methyl-4-(trifluoromethyl)-1*H*-imidazol-2-yl)benzyl)amino)pyrimidine-5-yl)dimethylphosphine oxide;
(2-(2-isopropylpyridin-3-yl)-4-((4-(1-methyl-4-(trifluoromethyl)-1*H*-imidazol-2-yl)benzyl)amino)pyrimidine-5-yl)dimethylphosphine oxide;
(4'-cyclopropyl-6'-(methoxy-$d_3$)-4-((4-(1-methyl-4-(trifluoromethyl)-1*H*-imidazol-2-yl)benzyl)amino)-[2,5'-bipyrimidine]-5-yl)dimethylphosphine oxide;
(2-(2-(difluoromethoxy)pyridin-3-yl)-4-((4-(1-methyl-4-(trifluoromethyl)-1*H*-imidazol-2-yl)benzyl)amino)pyrimidine-5-yl)dimethylphosphine oxide;
(4'-cyclopropyl-4-((3-fluoro-4-(1-methyl-4-(trifluoromethyl)-1*H*-imidazol-2-yl)benzyl)amino)-6'-methoxy-[2,5'-bipyrimidine]-5-yl)dimethylphosphine oxide;
(4'-cyclopropyl-4-((4-(1-cyclopropyl-4-(trifluoromethyl)-1*H*-imidazol-2-yl)benzyl)amino)-6'-methoxy-[2,5'-bipyrimidine]-5-yl)dimethylphosphine oxide;
(4'-cyclopropyl-6'-methoxy-4-((4-(5-methyl-3-(trifluoromethyl)-1*H*-pyrazole-1-yl)benzyl)amino)-[2,5'-bipyrimidine]-5-yl)dimethylphosphine oxide;
(4'-cyclopropyl-6'-methoxy-4-(((4-(1-methyl-4-(trifluoromethyl)-1*H*-imidazol-2-yl)bicyclo[2.2.2]octane-1-yl)methyl)amino)-[2,5'-bipyrimidine]-5-yl)dimethylphosphine oxide;
(4'-cyclopropyl-6'-methoxy-4-((((1*s*,2*R*,3*s*,4*r*,5*S*,6*r*,7*R*,8*S*)-4-(1-methyl-4-(trifluoromethyl)-1*H*-imidazol-2-yl)cuban-1-yl)methyl)amino)-[2,5'-bipyrimidine]-5-yl)dimethylphosphine oxide;
(4'-cyclopropyl-4-((4-(1-isopropyl-4-(trifluoromethyl)-1H-imidazol-2-yl)benzyl)amino)-6'-methoxy-[2,5'-bipyrimidine]-5-yl)dimethylphosphine oxide;
(4'-cyclopropyl-4-((3-fluoro-4-(1-isopropyl-4-(trifluoromethyl)-1*H*-imidazol-2-yl)benzyl)amino)-6'-methoxy-[2,5'-bipyrimidine]-5-yl)dimethylphosphine oxide;
(4'-cyclopropyl-4-((3-fluoro-4-(5-methyl-3-(trifluoromethyl)-1*H*-pyrazole-1-yl)benzyl)amino)-6'-methoxy-[2,5'-bipyrimidine]-5-yl)dimethylphosphine oxide;
(4'-cyclopropyl-4-((4-(5-isopropyl-3-(trifluoromethyl)-1*H*-pyrazole-1-yl)benzyl)amino)-6'-methoxy-[2,5'-bipyrimidine]-5-yl)dimethylphosphine oxide;
(4'-cyclopropyl-4-((3-fluoro-4-(5-isopropyl-3-(trifluoromethyl)-1*H*-pyrazole-1-yl)benzyl)amino)-6'-methoxy-[2,5'-bipyrimidine]-5-yl)dimethylphosphine oxide;
(4'-cyclopropyl-4-((4-(5-cyclopropyl-3-(trifluoromethyl)-1H-pyrazole-1-yl)benzyl)amino)-6'-methoxy-[2,5'-bipyrimidine]-5-yl)dimethylphosphine oxide;와
(4'-cyclopropyl-4-((4-(5-cyclopropyl-3-(trifluoromethyl)-1*H*-pyrazole-1-yl)-3-fluorobenzyl)amino)-6'-methoxy[2,5'-bipyrimidine]-5-yl)dimethylphosphine oxide.

The compound of the present invention can inhibit the activity of SOS1. More specifically, the compound of the present invention can block RAS activation by blocking the interaction between SOS1 and RAS.

Synthesis of the compound of the present invention

Compounds of the present invention, including Chemical Formula 1, can be synthesized by synthetic routes well known in the chemical arts, including those described herein and similar methods. Starting materials are generally available from commercial sources, such as Aldrich Chemicals (Milwaukee, Wisconsin), or are readily prepared using methods well known to those skilled in the art. For example, it can be prepared by methods generally described in the literature [Louis F. Fieser and Mary Fieser, Reagents for Organic Synthesis, 1st to 19th editions, Wiley, N.Y. (1967-1999)] or Beilsteins Handbuch der organischen Chemie, 4, Aufl (Springer-Verlag, Berlin, Germany)

For illustrative purposes, general methods for preparing the compounds and key intermediates of the present invention are outlined in the reaction schemes below. For more detailed descriptions of individual reaction steps, see the specific descriptions in the Examples below. Those skilled in the art will recognize that other synthetic routes can also be used to synthesize the compounds of the present invention. While specific starting materials and reagents are depicted in the reaction schemes and discussed below, other starting materials and reagents can be readily substituted to provide a variety of derivatives and/or reaction conditions. Furthermore, most compounds prepared by the methods described below can be further modified in light of the present disclosure using conventional chemistry well known to those skilled in the art.

The compounds of the present invention can use a form enriched with deuterium as a substituent or functional group. Such deuterium-modified forms can be obtained using the methods described in U.S. Patent Nos. 5,846,514 and 6,334,997, and can also be found in Dean, Dennis C., eds., <Recent Advances in the Synthesis and Applications of

Radiolabeled Compounds for Drug Discovery and Development>, published in Curr., Pharm. Des., 2000; 6 (10). Deuterium-modified starting materials are commercially available, and can be used in the synthetic methods described herein to obtain deuterium-substituted functional groups.

Pharmaceutical Composition

In another aspect, the present invention discloses a pharmaceutical composition comprising the aforementioned compound and a pharmaceutically acceptable excipient. The pharmaceutical composition of the present invention can be used to inhibit the guanine nucleotide exchange activity of SOS1 for RAS in the body, for example, for the treatment of cancer. In one embodiment, the pharmaceutical composition of the present invention can be used for the treatment of non-small cell lung cancer. In a more specific embodiment, the pharmaceutical composition of the present invention can be used for the treatment of cancer, including KRAS-mutant cancer.

In the pharmaceutical composition of the present invention, the aforementioned compounds may be present as pharmaceutically acceptable salts. Pharmaceutically acceptable salts include, for example, base addition salts and acid addition salts. Pharmaceutically acceptable base addition salts may be formed with metals or amines, such as alkali and alkaline earth metals or organic amines. Pharmaceutically acceptable salts of the compounds may also be prepared using pharmaceutically acceptable cations. Pharmaceutically acceptable acid addition salts include inorganic or organic acid salts. Examples of acid addition salts include those derived from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, benzoic acid, hydroxybenzoic acid, phenylacetic acid, cinnamic acid, salicylic acid, methanesulfonic acid, ethanesulfonic acid, benzenesulfonic acid, or toluenesulfonic acid.

In the present invention, a pharmaceutically acceptable excipient refers to an inactive ingredient approved by a relevant administrative agency (e.g., the Ministry of Food and Drug Safety of the Republic of Korea, the Federal Food and Drug Administration (FDA) of the United States) as being suitable for use together with an active pharmaceutical ingredient for the purpose of manufacturing a medicine for treating a disease in humans or livestock. Such pharmaceutically acceptable excipients include, but are not limited to, carriers, lubricants, fluidizing agents, disintegrating agents, sweeteners, diluents, preservatives, coloring agents, flavoring agents, surfactants, wetting agents, dispersing agents, suspending agents, stabilizers, isotonic agents, solvent emulsifiers, and adjuvants.

The pharmaceutical composition of the present invention may be in a form suitable for oral administration, such as a tablet, capsule, pill, powder, sustained-release preparation, solution, or suspension; in a form suitable for parenteral injection, such as a sterile solution, suspension, or emulsion; in a form suitable for topical administration, such as an ointment or cream; or in a form suitable for rectal administration, such as a suppository.

The pharmaceutical composition according to the present invention can be prepared in a conventional manner, for example, by conventional mixing, dissolving, granulating, sugar-coated tablet preparation, powdering, emulsifying, encapsulating, encapsulating, or lyophilizing processes. The appropriate formulation will vary depending on the chosen route of administration.

Pharmaceutical compositions suitable for oral administration can be readily formulated by combining the compounds disclosed herein with pharmaceutically acceptable excipients, such as carriers well known in the art. Using such excipients and carriers, the compounds disclosed herein can be formulated as tablets, pills, sugar-coated tablets, capsules, solutions, gels, syrups, slurries, suspensions, and the like for oral ingestion by patients to be treated. Oral pharmaceutical preparations can be obtained by adding the compounds of the present invention together with solid excipients, grinding the resulting mixture if necessary, adding suitable auxiliaries if necessary, and then processing the granulated mixture to form cores of tablets or sugar-coated tablets. Suitable excipients include, for example, fillers and cellulose preparations. If desired, a disintegrant may be added.

For pharmaceutical compositions for oral administration of a therapeutically effective amount of a compound of the present disclosure, the compositions are generally in the form of solid (e.g., tablets, capsules, pills, powders, or troches) or liquid formulations (e.g., aqueous suspensions, solutions, elixirs, or syrups).

When administered in tablet form, the composition may additionally contain functional solids and/or solid carriers such as gelatin or adjuvants. Compositions in the form of tablets, capsules, and powders may contain from about 1 to about 95 wt% of the compound of the present invention, preferably from about 15 to about 90 wt% of the compound of the present invention, based on the total weight of the composition. An example of a tablet composition may contain, for example, up to about 80 wt% of the active pharmaceutical ingredient, from about 10 to about 90 wt% of a binder, from about 0 to about 85 wt% of a diluent, from about 2 to about 10 wt% of a disintegrant, and from about 0.25 to about 10 wt% of a lubricant.

When administered in liquid or suspension form, a functional liquid and/or liquid carrier such as water, petroleum, or animal or vegetable oil may be added. Liquid compositions may further contain saline, sugar alcohol solutions, dextrose or other sugar solutions, or glycols. When administered in liquid or suspension form, the composition may contain from about 0.5 to about 90% by weight of the compound of the present invention, preferably from about 1 to about 50% by weight

of the compound of the present invention. In one contemplated embodiment, the liquid carrier is non-aqueous or substantially non-aqueous. For administration in liquid form, the composition may be supplied as a rapidly dissolving solid formulation for dissolution or suspension immediately prior to administration.

When the pharmaceutical composition of the present invention is administered intravenously, transdermally, or subcutaneously, it is in the form of a parenteral aqueous solution that does not contain a pyrogen. The preparation of such parenteral solutions by considering pH, isotonicity, stability, etc., is within the skill of the art. Preferred compositions for intravenous, transdermal, or subcutaneous injection generally contain an isotonic vehicle. Such compositions can be prepared for administration as a solution of the free base or a pharmacologically acceptable salt in water suitably mixed with a surfactant such as hydroxypropyl cellulose. Dispersions in glycerol, liquid polyethylene glycol, and mixtures thereof, as well as dispersions in oils, can also be prepared. Under normal storage and use conditions, such preparations may optionally contain a preservative to prevent the growth of microorganisms.

Injectable compositions may include sterile aqueous solutions, suspensions, or dispersions, and sterile powders for the extemporaneous preparation of sterile injectable solutions, suspensions, or dispersions. Sterile injectable solutions are prepared by incorporating the active compound in the required amount in an appropriate solvent with various other ingredients, as required, followed by filtered sterilization. Generally, dispersions are prepared by incorporating the various sterile active ingredients into a sterile vehicle that contains a basic dispersion medium and the required other ingredients from those enumerated above. In embodiments of sterile powders for the preparation of sterile injectable solutions, the preferred methods of preparation are vacuum drying and freeze-drying techniques, which produce a powder of the active ingredient and any additional required ingredients from a previously sterile-filtered solution thereof.

To achieve controlled release of the active compound upon contact with body fluids in the gastrointestinal tract and to provide substantially constant and effective levels of the active compound in the plasma, sustained-release or sustained-release formulations may be prepared. For example, release may be controlled by one or more of dissolution, diffusion, and ion exchange. Furthermore, sustained-release approaches may enhance absorption through saturation or restriction pathways within the gastrointestinal tract. For example, for this purpose, the compound may be embedded in a polymer matrix comprising a biodegradable polymer, a water-soluble polymer, or a mixture thereof, and optionally a suitable surfactant. Embedding in this context may mean incorporating microparticles into the polymer matrix. Controlled-release formulations may also be obtained by encapsulating dispersed microparticles or emulsified microdroplets using known dispersion or emulsion coating techniques.

The pharmaceutical composition of the present invention may be formulated for parenteral administration by injection (e.g., bolus injection or continuous infusion). The injectable formulation may be presented in unit dosage form (e.g., ampoules or multi-dose containers) with an added preservative. The composition may take the form of a suspension, solution, or emulsion in an oily or aqueous vehicle, and may contain formulating agents such as suspending, stabilizing, and/or dispersing agents.

Pharmaceutical compositions may be presented in unit dosage forms suitable for single-dose administration of precise dosages.

Pharmaceutical compositions containing the compounds of the present invention may be used according to the methods described below.

Method of Treatment

In another aspect, the present invention provides a method of treating a disease requiring inhibition of SOS1 activity. This method comprises administering a therapeutically effective amount of a compound of the present invention, such as a compound defined by Formula 1, or a pharmaceutical composition comprising such a compound of the present invention, to a patient requiring inhibition of SOS1 activity.

The diseases requiring inhibition of SOS1activity include cancer.

In one embodiment, the present invention provides a method for treating cancer. The method comprises administering to a cancer patient a therapeutically effective amount of a compound of the present invention, such as a compound defined by Formula 1, or a pharmaceutical composition comprising such a compound of the present invention. In one specific embodiment, the cancer is a KRAS G12C, G12D, G12V, or G13D mutant cancer.

In a method for treating cancer, the compound of the present invention may be administered alone or in combination with at least one other drug. These other drugs and the compound of the present invention may be administered simultaneously or sequentially. Some examples of drugs that may be administered in combination with the drug of the present invention include RAS inhibitors, MEK inhibitors, and EGFR inhibitors. Specific examples of RAS inhibitors include sotorasib and adagrasib. Specific examples of MEK inhibitors include trametinib, cobimetinib, selumetinib, and binimetinib. Specific examples of EGFR inhibitors include gefitinib, erlotinib, afatinib, brigatinib, osimertinib, cetuximab, and panitumumab.

As used herein, unless otherwise specified, a "therapeutically effective amount" of a compound refers to an amount of the compound sufficient to delay or minimize one or more symptoms associated with a disease, disorder, or

condition, or to provide a therapeutic effect against a disease, disorder, or condition. The term "therapeutically effective amount" encompasses an amount that improves overall healing, an amount that alleviates or avoids symptoms or causes of the disease or condition, and an amount that enhances the therapeutic efficacy of other treatments.

The amount of the compound administered may vary depending on the subject being treated, the subject's age, health, sex, and weight, the type of concurrent treatment (if any), the severity of the pain, the nature of the desired effect, the mode and frequency of treatment, and the judgment of the prescribing physician. The frequency of administration may also vary depending on the pharmacodynamic effects on arterial oxygen pressure. However, the most desirable dosage can be tailored to the individual subject, as understood and determined by one of skill in the art without undue experimentation. This usually involves adjusting the standard dose (e.g., reducing the dose if the patient is underweight).

Although individual case needs may vary, determining the optimal range of therapeutically effective doses of a compound is common sense in this field. When administered to humans in the therapeutic or prophylactic treatment of conditions and disorders using the compounds of the present invention, for example, a typical dosage of the compounds of the present invention may be from about 0.05 mg/kg/day to about 50 mg/kg/day, for example, at least 0.05 mg/kg, at least 0.08 mg/kg, at least 0.1 mg/kg, at least 0.2 mg/kg, at least 0.3 mg/kg, at least 0.4 mg/kg, or at least 0.5 mg/kg, preferably not more than 50 mg/kg, not more than 40 mg/kg, not more than 30 mg/kg, not more than 20 mg/kg, or not more than 10 mg/kg, which may be, for example, from about 2.5 mg/day (0.5 mg/kg x 5 kg) to about 5000 mg/day (50 mg/kg x 100 kg). For example, the dosage of the compound may be about 0.1 mg/kg/day to about 50 mg/kg/day, about 0.05 mg/kg/day to about 10 mg/kg/day, about 0.05 mg/kg/day to about 5 mg/kg/day, about 0.05 mg/kg/day to about 3 mg/kg/day, about 0.07 mg/kg/day to about 3 mg/kg/day, about 0.09 mg/kg/day to about 3 mg/kg/day, about 0.05 mg/kg/day to about 0.1 mg/kg/day, about 0.1 mg/kg/day to about 1 mg/kg/day, about 1 mg/kg/day to about 10 mg/kg/day, about 1 mg/kg/day to about 5 mg/kg/day, about 1 mg/kg/day to about 3 mg/kg/day, about 3 mg/day to about The dose may be 500 mg/day, about 5 mg/day to about 250 mg/day, about 10 mg/day to about 100 mg/day, about 3 mg/day to about 10 mg/day, or about 100 mg/day to about 250 mg/day. These doses may be administered as a single dose or divided into multiple doses.

In another aspect of the present invention, a method for inhibiting intracellular activity of SOS1 is provided. The method comprises contacting a cell in which inhibition of SOS1 activity is desired, with an effective amount of a compound of the present invention, for example, a compound defined by Chemical Formula 1.

[EXAMPLE]

Hereinafter, the present invention will be described in more detail through the following examples and experimental examples. However, these examples and experimental examples are intended only to aid understanding of the present invention and are not intended to limit the scope of the present invention in any way. Various changes and modifications may be made to the present examples, and such changes and modifications also fall within the scope of the appended claims.

The full names of the abbreviations used in the synthetic methods below are as follows:

Ph: phenyl
TEA: triethylamine
THF: tetrahydrofuran
$Pd_2(dba)_3$: tris(dibenzylideneacetone)dipalladium
XantPhos: (9,9-dimethyl-9H-xanthene-4,5-diyl)bis(diphenylphosphane)

**[Example 1]**

**Preparation of (R)-6,7-dimethoxyl-2-methyl-N-(1-(4-(2-((methylamino)methyl)phenyl)selenophen-2-yl)ethyl) quinazolin-4-amine**

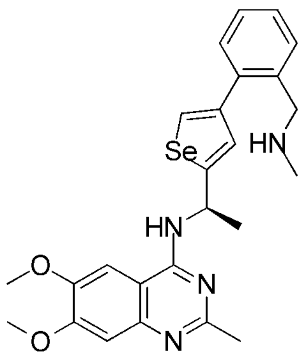

The compound of Example 1 was prepared by the reaction scheme below.

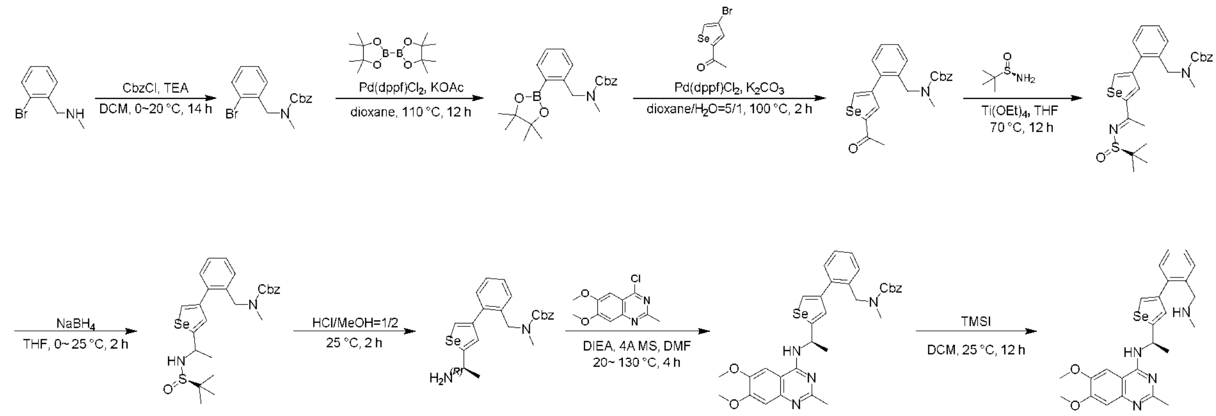


Step (1) Preparation of benzyl (2-bromobenzyl)(methyl)carbamate

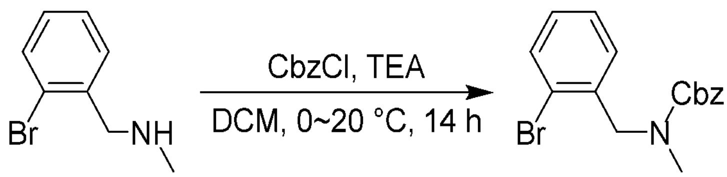


1-(2-Bromophenyl)-N-methylmethanamine (5.00 g, 24.9 mmol, 1.00 eq) was dissolved in dichloromethane (50.0 mL), and then triethylamine (5.06 g, 49.9 mmol, 6.96 mL, 2.00 eq) and benzyl carbonochloridate (4.69 g, 27.4 mmol, 3.92 mL, 1.10 eq) were added at 0 °C. The mixture was stirred at 20 °C for 2 h. At 0 °C, triethylamine (2.53 g, 24.9 mmol, 3.48 mL, 1.00 eq) and benzyl carbonochloridate (2.13 g, 12.5 mmol, 1.78 mL, 0.500 eq) were added to the mixture, and the mixture was stirred at 20 °C for 12 h. The mixture was concentrated under reduced pressure to obtain a residue, which was purified by column chromatography ($SiO_2$, petroleum ether/ethyl acetate = 100/1 to 0/1) to give benzyl (2-bromobenzyl)(methyl) carbamate (7.80 g, 23.3 mmol, 93% yield).

$^1H$ NMR (400 MHz, $CDCl_3$) $\delta$ = 7.56 (d, *J* = 7.2 Hz, 1H), 7.45 - 7.20 (m, 7H), 7.17 - 7.12 (m, 1H), 5.20 (br d, *J* = 16.4 Hz, 2H), 4.62 (br d, *J* = 18.4 Hz, 2H), 2.96 (br d, *J* = 11.6 Hz, 3H).

Step (2) Preparation of benzyl methyl(2-(4,4,5,5-tetramethyl-1,3,2-dioxaborolane-2-yl)benzyl)carbamate.

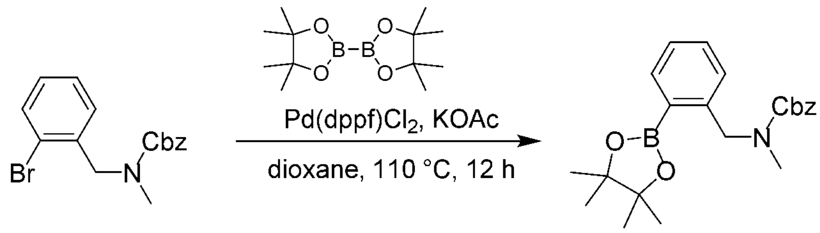


Benzyl (2-bromobenzyl)(methyl)carbamate (2.00 g, 5.98 mmol, 1.00 eq) was dissolved in dioxane (30.0 mL), and 4,4,4',4',5,5,5',5'-octamethyl-2,2'-bi(1,3,2-dioxoborolane) (2.28 g, 8.98 mmol, 1.50 eq), potassium acetate (1.47 g, 14.9 mmol, 2.50 eq), and [1,1-bis(diphenylphosphino)ferrocene]dichloropalladium(II) (437 mg, 598 μmol, 0.100 eq) were added. The mixture was stirred at 110 °C for 12 h under a nitrogen atmosphere. The mixture was filtered, and the filtrate was concentrated under reduced pressure to obtain a residue. The residue was purified by column chromatography ($SiO_2$, petroleum ether/ethyl acetate=100/1 to 0/1) to obtain benzyl methyl(2-(4,4,5,5-tetramethyl-1,3,2-dioxaborolan-2-yl) benzyl)carbamate (2.40 g, 5.67 mmol, 94% yield, 90% purity) as a green oil.

$^1H$ NMR (400 MHz, $CDCl_3$) $\delta$ = 7.83 (br d, *J* = 7.2 Hz, 1H), 7.45 - 7.27 (m, 7H), 7.26 - 7.19 (m, 1H), 5.25 - 5.14 (m, 2H), 4.86 (br d, *J* = 8.8 Hz, 2H), 2.90 (br d, *J* = 12.8 Hz, 3H), 1.34 (s, 12H).

<u>Step (3)</u> Preparation of benzyl (2-(5-acetylselenophen-3-yl)benzyl)(methyl)carbamate

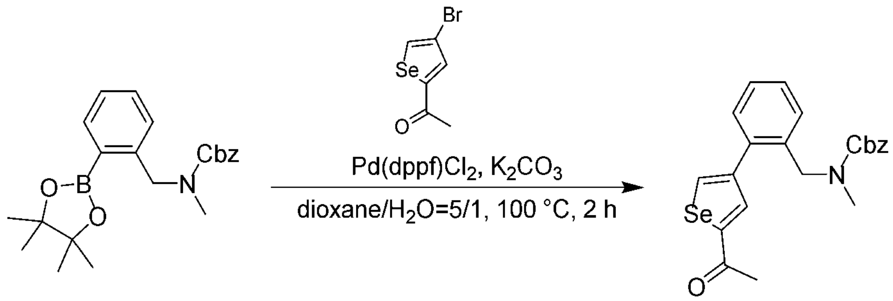


1-(4-Bromoselenophen-2-yl)ethan-1-one (1.10 g, 4.37 mmol, 1.00 eq) and benzyl methyl(2-(4,4,5,5-tetramethyl-1,3,2-dioxoborolan-2-yl)benzyl)carbamate (2.22 g, 5.24 mmol, 1.20 eq) were dissolved in dioxane (15.0 mL) and water (3.00 mL), and then [1,1-bis(diphenylphosphino)ferrocene]dichloropalladium(II) (319 mg, 436 μmol, 0.100 eq) and potassium carbonate (1.81 g, 13.1 mmol, 3.00 eq) were added. The mixture was stirred at 100 °C for 2 h under a nitrogen atmosphere. The mixture was filtered, and the filtrate was concentrated under reduced pressure to obtain a residue. The residue was purified by column chromatography ($SiO_2$, petroleum ether/ethyl acetate=100/1 to 0/1) and then lyophilized to obtain benzyl (2-(5-acetylselenophen-3-yl)benzyl)(methyl)carbamate (1.15 g, 2.70 mmol, 61% yield) as a pale yellow oil.

$^1H$ NMR (400 MHz, $CDCl_3$) $\delta$ = 8.27 - 7.97 (m, 1H), 7.96 - 7.74 (m, 1H), 7.42 - 7.28 (m, 8H), 7.25 - 7.19 (m, 1H), 5.20 - 5.01 (m, 2H), 4.62 - 4.42 (m, 2H), 2.89 - 2.71 (m, 3H), 2.64 - 2.46 (m, 3H).

<u>Step (4)</u> Preparation of benzyl (*R,E*)-(2-(5-(1-((*tert*-butylsulfinyl)imino)ethyl)selenophen-3-yl)benzyl)(methyl)carbamate

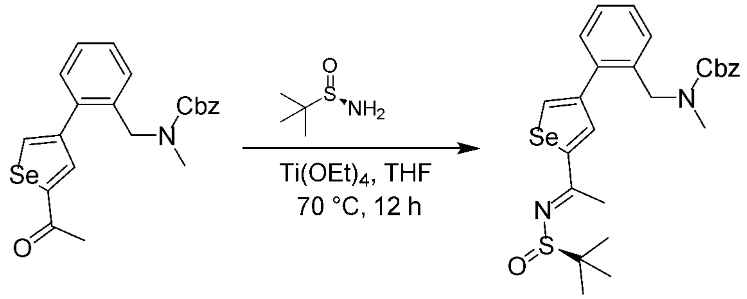

Benzyl (2-(5-acetylselenophen-3-yl)benzyl)(methyl)carbamate (300 mg, 703 μmol, 1.00 eq) was dissolved in tetrahydrofuran (6.00 mL), and then (R)-2-methylpropane-2-sulfinylamide (255 mg, 2.11 mmol, 3.00 eq) and titanium(IV) ethoxide (481 mg, 2.11 mmol, 437 μL, 3.00 eq) were added. The mixture was stirred at 70 °C for 12 h. The mixture was concentrated under reduced pressure to obtain a residue. The residue was purified by column chromatography ($SiO_2$, petroleum ether/ethyl acetate=100/1 to 0/1) to give benzyl (R,E)-(2-(5-(1-((tert-butylsulfinyl)imino)ethyl)selenophen-3-yl) benzyl)(methyl)carbamate (270 mg, 489 μmol, 69% yield, 96% purity) as a yellow oil.

$^1H$ NMR (400 MHz, $CDCl_3$) $\delta$ = 8.04 - 7.86 (m, 1H), 7.79 - 7.54 (m, 1H), 7.42 - 7.27 (m, 8H), 7.25 - 7.20 (m, 1H), 5.12 (br d, *J* = 17.2 Hz, 2H), 4.62 - 4.44 (m, 2H), 2.92 - 2.63 (m, 6H), 1.32 (s, 9H).

<u>Step (5)</u> Preparation of benzyl (2-(5-(1-(((*R*)-*tert*-butylsulfinyl)amino)ethyl)selenophen-3-yl)benzyl)(methyl)carbamate

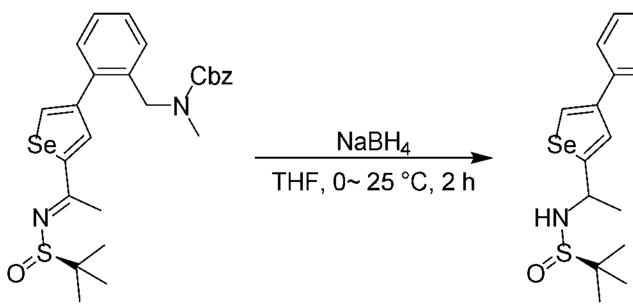


Benzyl (R,E)-(2-(5-(1-((tert-butylsulfinyl)imino)ethyl)selenophen-3-yl)benzyl)(methyl)carbamate (270 mg, 509 μmol, 1.00 eq) was dissolved in tetrahydrofuran (4.00 mL), and then sodium borohydride (57.8 mg, 1.53 mmol, 3.00 eq) was added at 0 °C. The mixture was stirred at 25 °C for 2 h. The mixture was poured into ice-water (10 mL), extracted with ethyl acetate (3 × 10 mL), washed with brine (20 mL), and dried over anhydrous sodium sulfate. The residue was then filtered and concentrated under reduced pressure to obtain the residue. The residue was purified by column chromatography ($SiO_2$, petroleum ether/ethyl acetate=100/1 to 0/1) to give benzyl (2-(5-(1-(((R)-tert-butylsulfinyl)amino)ethyl) selenophen-3-yl)benzyl)(methyl)carbamate (220 mg, 393 μmol, 77% yield, 95% purity) as a colorless oil.

$^1H$ NMR (400 MHz, $CDCl_3$) $\delta$ = 7.72 - 7.62 (m, 1H), 7.40 - 7.27 (m, 8H), 7.25 - 7.12 (m, 2H), 5.14 (br d, *J* = 8.0 Hz, 2H), 4.84 (br s, 1H), 4.63 - 4.47 (m, 2H), 2.90 - 2.67 (m, 3H), 1.61 (br d, *J* = 6.4 Hz, 3H), 1.27 (s, 9H).

<u>Step (6)</u> Preparation of benzyl (*R*)-(2-(5-(1-aminoethyl)selenophen-3-yl)benzyl)(methyl)carbamate

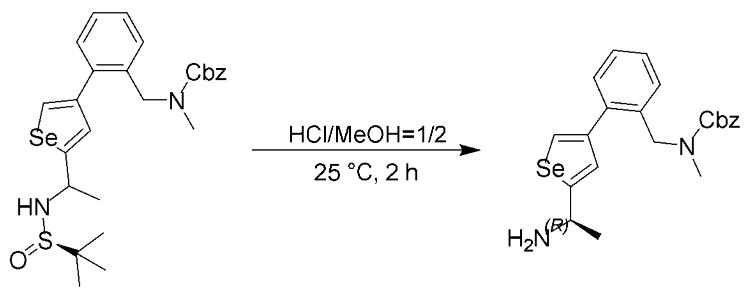


Benzyl (R)-(2-(5-(1-aminoethyl)selenophen-3-yl)benzyl)(methyl)carbamate (220 mg, 351 μmol, 1.00 eq) was dissolved in methanol (2.00 mL), and then concentrated hydrochloric acid (1.00 mL) was added. The mixture was stirred at 25 °C for 2 h. The pH of the mixture was adjusted to 7 by adding sodium hydroxide solution (2 N) at 0 °C, extracted with a mixture of dichloromethane and isopropanol (3:1) (3 × 20 mL), and dried over anhydrous sodium sulfate. After filtration, the mixture was concentrated under reduced pressure to obtain benzyl (R)-(2-(5-(1-aminoethyl)selenophen-3-yl)benzyl) (methyl)carbamate (200 mg, raw material) as a yellow oil.

$^1H$ NMR (400 MHz, DMSO-$d_6$) $\delta$ = 8.08 - 7.91 (m, 1H), 7.44 - 7.24 (m, 9H), 7.17 (br d, *J* = 7.6 Hz, 3H), 5.17 - 4.97 (m, 2H), 4.65 - 4.54 (m, 1H), 4.50 (s, 2H), 2.77 (s, 3H), 1.51 (br s, 3H).

Step (7) Preparation of benzyl (R)-(2-(5-(1-((6,7-dimethoxy-2-methylquinazolin-4-yl)amino)ethyl)selenophen-3-yl)benzyl)(methyl) carbamate

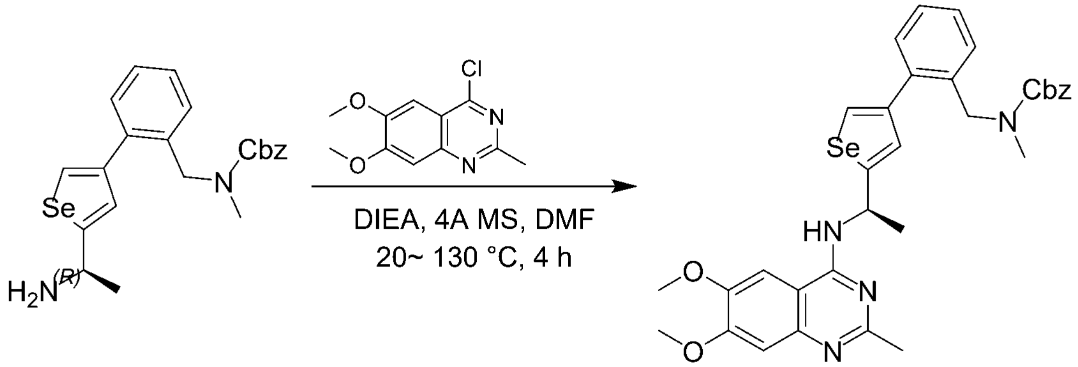


Benzyl (R)-(2-(5-(1-aminoethyl)selenophen-3-yl)benzyl)(methyl)carbamate (75.0 mg, 175 μmol, 1.00 eq) was dissolved in dimethylformamide (1.50 mL), and then 4-chloro-6,7-dimethoxy-2-methylquinazoline (41.8 mg, 175 μmol, 1.00 eq), N,N-diisopropylethylamine (68.0 mg, 526 μmol, 91.7 μL, 3.00 eq), and 4A MS (225 mg) were added. The mixture was stirred at 20 °C for 0.5 h. The mixture was then stirred at 130 °C for 3.5 h. After filtration of the mixture, the filtrate was purified by Prep-HPLC (Column: Waters Xbridge 150□25mm□5□m; Mobile phase: [water(zinc carbonate)-acetonitrile]; Gradient: 58%-88% B, for 9 min) to obtain benzyl (R)-(2-(5-(1-((6,7-dimethoxy-2-methylquinazolin-4-yl)amino)ethyl) selenophen-3-yl)benzyl)(methyl)carbamate (74.0 mg, 116 μmol, 33% yield, 99% purity) as a yellow solid.

$^1H$ NMR (400 MHz, DMSO-$d_6$) $\delta$ = 8.12 (br d, $J$ = 7.6 Hz, 1H), 7.89 - 7.77 (m, 1H), 7.62 (s, 1H), 7.40 - 7.24 (m, 8H), 7.16 (br d, $J$ = 7.2 Hz, 2H), 7.05 (s, 1H), 5.95 - 5.78 (m, 1H), 5.14 - 4.96 (m, 2H), 4.49 (s, 2H), 3.87 (d, $J$ = 4.0 Hz, 6H), 2.75 (br s, 3H), 2.44 (s, 3H), 1.72 (br s, 3H).

Step (8) Preparation of (R)-6,7-dimethoxyl-2-methyl-N-(1-(4-(2-((methylamino)methyl)phenyl)selenophen-2-yl)ethyl) quinazolin-4-amine

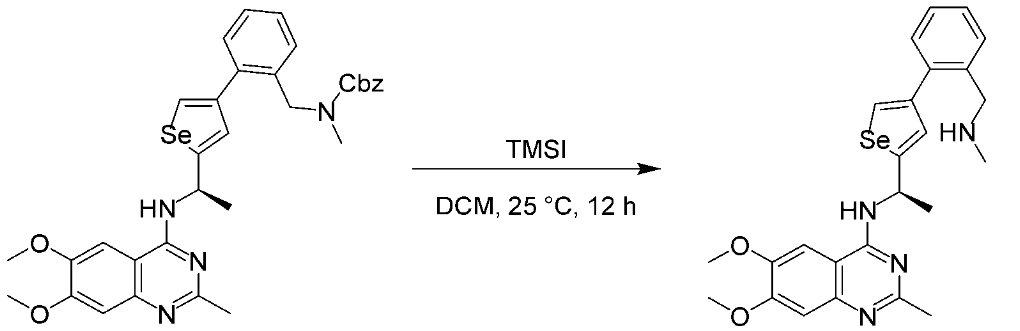


Benzyl (R)-(2-(5-(1-((6,7-dimethoxy-2-methylquinazolin-4-yl)amino)ethyl)selenophen-3-yl)benzyl)(methyl) carbamate (40 mg, 63.5 μmol, 1.00 eq) was dissolved in dichloromethane (1.00 mL), and then iodotrimethylsilane (38.1 mg, 191 μmol, 25.9 μL, 3.00 eq) was added. The mixture was stirred at 25 °C for 12 h. Then, 2 mL of methanol was added, and the resulting mixture was stirred at room temperature for an additional 30 min and concentrated under reduced pressure. The residue was purified by Prep-HPLC (Column: Waters Xbridge 150 x 25mm x 5μm; Mobile phase: [water(zinc carbonate)-acetonitrile]; Gradient: 32%-62% B, for 9 min) to give (R)-6,7-dimethoxyl-2-methyl-N-(1-(4-(2-((methylamino)methyl)phenyl)selenophen-2-yl)ethyl)quinazolin-4-amine (11.0 mg, 22.1 μmol, 35% yield, 99% purity) as a white solid.

$^1H$ NMR (400 MHz, DMSO-$d_6$) $\delta$ = 8.15 (d, $J$ = 8.1 Hz, 1H), 7.97 (d, $J$ = 1.3 Hz, 1H), 7.65 (s, 1H), 7.49 - 7.42 (m, 2H), 7.33 - 7.23 (m, 3H), 7.06 (s, 1H), 5.91 (m, 1H), 3.88 (s, 6H), 3.57 (s, 2H), 2.46 (s, 3H), 2.23 (s, 3H), 1.74 (d, $J$ = 7.0 Hz, 3H).

**[Example 2]**

**Preparation of 7-methoxy-2-methyl-*N*-((*R*)-1-(4-(2-((methylamino)methyl)phenyl)selenophen-2-yl) ethyl)-6-(((S)-tetrahydrofuran-3-yl)oxy)quinazolin-4-amine.**

The compound of Example 2 was prepared according to the reaction scheme below.

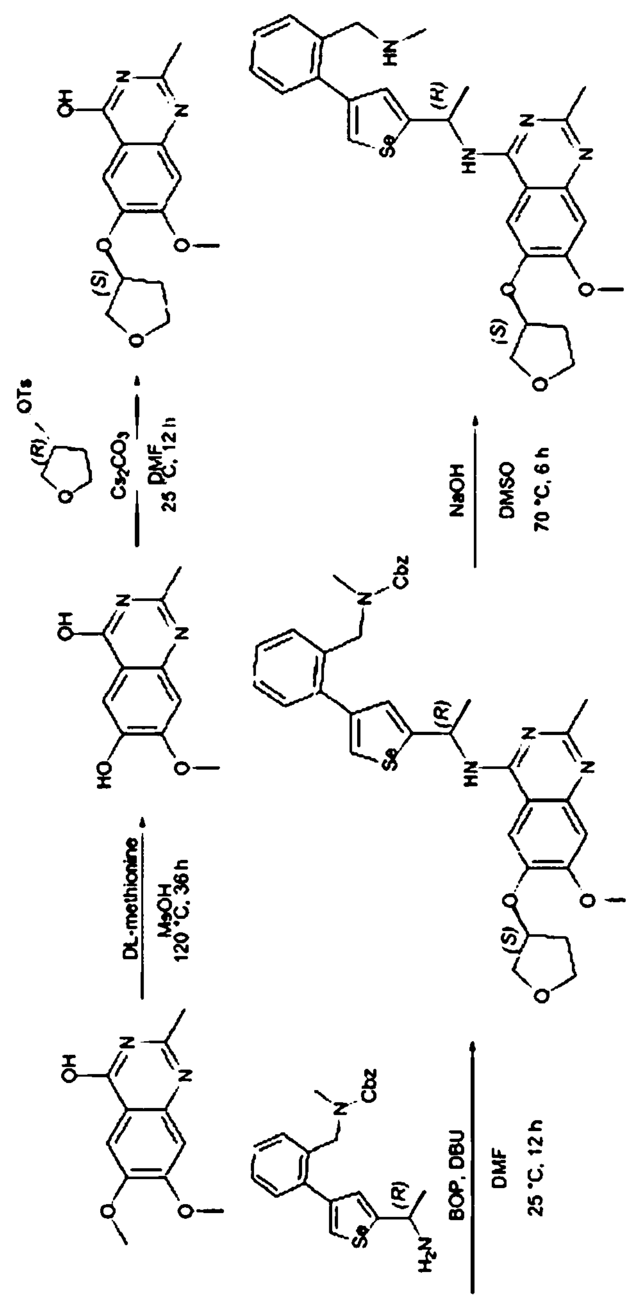


Step (1) 7-methoxy-2-methylquinazolin-4,6-diol

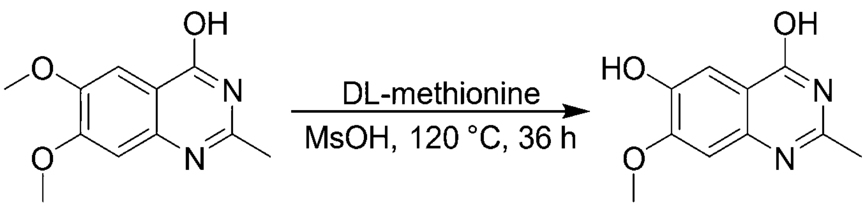

6,7-Dimethoxyl-2-methylquinazolin-4-ol (4.80 g, 21.8 mmol, 1.00 eq) was dissolved in methanesulfonic acid (25.0 mL), and DL-methionine (4.88 g, 32.7 mmol, 1.50 eq) was added. The mixture was stirred at 120 °C for 36 h. The reaction mixture was poured into ice water (500 mL) and basified with soda water until the pH became 9. The solution was filtered to obtain a filter cake. The filter cake was triturated with 3 M hydrochloric acid at 25 °C for 12 h. The obtained mixture was filtered, and the filter cake was dried under vacuum to obtain 7-methoxy-2-methylquinazolin-4,6-diol (2.40 g, 11.6 mmol, 53% yield) as a yellow solid.

$^{1}$H NMR (400 MHz, DMSO-$d_6$) $\delta$ = 9.67 (s, 1H), 7.32 (s, 1H), 7.01 (s, 1H), 3.87 (s, 3H), 2.28 (s, 3H).

Step (2) Preparation of (S)-7-methoxy-2-methyl-6-((tetrahydrofuran-3-yl)oxy)quinazolin-4-ol

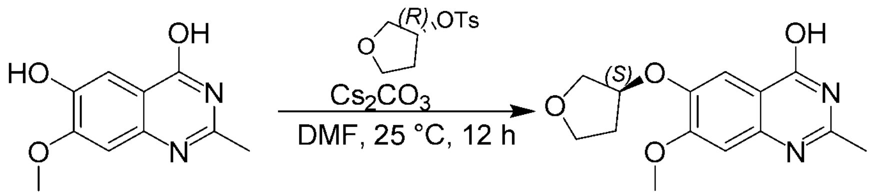


7-Methoxy-2-methylquinazolin-4,6-diol (1.20 g, 5.82 mmol, 1.00 eq) was dissolved in dimethylformamide (50.0 mL), and (R)-tetrahydrofuran-3-yl 4-methylbenzenesulfonate (1.41 g, 5.82 mmol, 1.00 eq) and cesium carbonate (2.84 g, 8.73 mmol, 1.50 eq) were added. The mixture was stirred at 25 °C for 12 h. The reaction mixture was filtered to obtain a filtrate. The filtrate was concentrated in vacuo to obtain a residue. The residue was purified by Prep-HPLC (Column: Waters Xbridge Prep OBD C18 150□40 mm□10 □m; Mobile phase: [Water (ammonium sulfite) -Acetonitrile]; Gradient: 0%- 30% B for 15 min) to give (S)-7-methoxy-2-methyl-6-((tetrahydrofuran-3-yl)oxy)quinazolin-4-ol (800 mg, 2.90 mmol, 49.8% yield) as a white solid.

$^{1}$H NMR (400 MHz, DMSO-$d_6$) $\delta$ = 7.34 (s, 1H), 7.04 (s, 1H), 5.08 (br d, *J = 1.2* Hz, 1H), 3.91 - 3.73 (m, 7H), 2.29 (s, 3H), 2.27 - 2.18 (m, 1H), 2.04 - 1.95 (m, 1H).

Step (3) Preparation of benzyl (2-(5-((R)-1-((7-methoxy-2-methyl-6-(((S)-tetrahydrofuran-3-yl)oxy)quinazolin-4-yl)amino)ethyl) selenophen-3-yl)benzyl)(methyl)carbamate

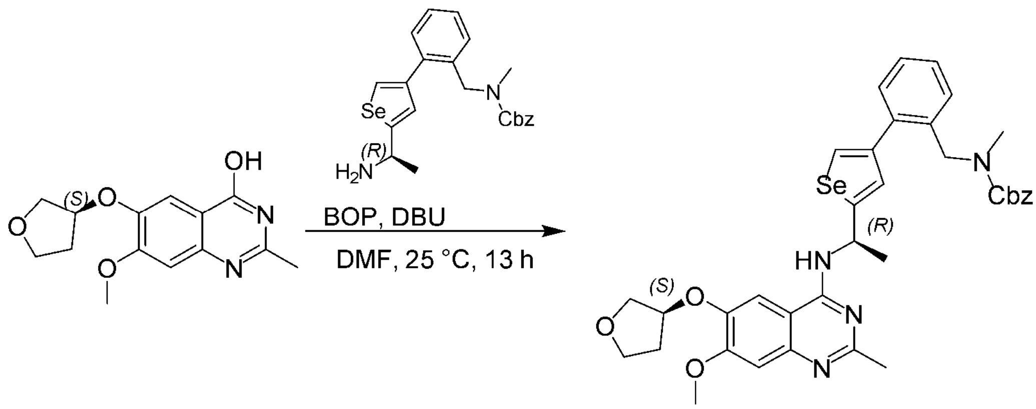


(S)-7-methoxy-2-methyl-6-((tetrahydrofuran-3-yl)oxy)quinazolin-4-ol (200 mg, 724 μmol, 1.00 eq), ((1H-benzo [d][1,2,3]triazol-1-yl)oxy)tris(dimethylamino)phosphonium hexafluorophosphate (V) (416 mg, 941 μmol, 1.30 eq), and 2,3,4,6,7,8,9,10-octahydropyrimido[1,2-a]azepine (548 mg, 3.60 mmol, 543 μL, 4.97 eq) were dissolved in dimethylformamide (10.0 mL) and stirred at 25°C for 1 h. Afterwards, benzyl (R)-(2-(5-(1-aminoethyl)selenophen-3-yl)benzyl) (methyl)carbamate (309 mg, 724 μmol, 1.00 eq) was added, and the mixture was stirred at 25°C for 12 h. The mixture was concentrated in vacuo to obtain a residue. The residue was purified by Prep-HPLC (column: YMC-Actus Triart C18 150 x 30 mm x 7 μm; mobile phase: [water (formic acid)-acetonitrile]; gradient: 33%- 63% B for 10 min) to give benzyl (2-(5-((R)-1-((7-methoxy-2-methyl-6-(((S)-tetrahydrofuran-3-yl)oxy)quinazolin-4-yl)amino)ethyl) selenophen-3-yl)benzyl)(methyl)carbamate (54.0 mg, 78.8 μmol, 10.9% yield) as a yellow solid.

$^1$H NMR (400 MHz, DMSO-$d_6$) $\delta$ = 14.17 - 13.92 (m, 1H), 9.80 (br dd, *J* = 2.4, 3.6 Hz, 1H), 8.03 - 7.93 (m, 1H), 7.39 - 7.23 (m, 8H), 7.18 (br d, *J* = 7.2 Hz, 1H), 7.14 - 7.05 (m, 2H), 6.12 - 6.00 (m, 1H), 5.18 - 5.12 (m, 1H), 5.08 - 4.93 (m, 2H), 4.49 (s, 2H), 3.97 (s, 4H), 3.87 - 3.77 (m, 3H), 2.76 (s, 3H), 2.63 (br s, 3H), 2.03 - 1.96 (m, 2H), 1.78 (br s, 3H).

Step (4) Preparation of 7-methoxy-2-methyl-N-((R)-1-(4-(2-((methylamino)methyl)phenyl)selenophen-2-yl)ethyl)-6-(((*S*)-tetrahydrofuran-3-yl)oxy)quinazolin-4-amine

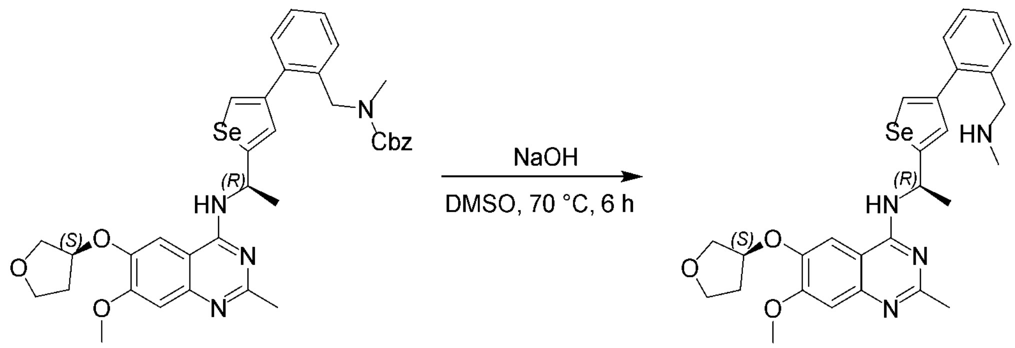


Benzyl (2-(5-((R)-1-((7-methoxy-2-methyl-6-(((S)-tetrahydrofuran-3-yl)oxy)quinazolin-4-yl)amino)ethyl)selenophen-3-yl)benzyl)(methyl)carbamate (45.0 mg, 65.6 μmol, 1.00 eq) was dissolved in dimethyl sulfoxide (2.00 mL), and then sodium hydroxide (13.1 mg, 328 μmol, 5.00 eq) was added. The mixture was stirred at 70°C for 6 h. The mixture was purified by Prep-HPLC (column: YMC-Actus Triart C18 150 x 30 mm x 7 μm; mobile phase: [water (formic acid)-acetonitrile]; gradient: 8%-38% B for 10 min) and lyophilized to obtain 7-methoxy-2-methyl-N-((R)-1-(4-(2-((methylamino)methyl)phenyl)selenophen-2-yl)ethyl)-6-(((S)-tetrahydrofuran-3-yl)oxy)quinazolin-4-amine (10.56 mg, 18.27 μmol, 27.8% yield, 95.4% purity) as a brown solid.

$^1$H NMR (400 MHz, DMSO-$d_6$) $\delta$ = 8.27 (s, 1H), 8.19 (br d, *J* = 8.4 Hz, 1H), 7.95 (s, 1H), 7.67 - 7.61 (m, 1H), 7.54 (br d, *J* = 6.4 Hz, 1H), 7.40 (s, 1H), 7.37 - 7.29 (m, 3H), 7.07 (s, 1H), 5.90 (br t, *J* = 7.2 Hz, 1H), 5.20 - 5.08 (m, 1H), 3.96 (br dd, J = 4.4, 10.0 Hz, 1H), 3.87 (s, 3H), 3.84 - 3.75 (m, 6H), 2.45 (s, 3H), 2.34 (s, 3H), 2.29 (br s, 1H), 1.97 (br s, 1H), 1.74 (d, *J* = 6.8 Hz, 3H).

**[Example 3]**

**Preparation of (R)-(2-chloro-4-((1-(4-(2-((methylamino)methyl)phenyl)selenophen-2-yl)ethyl)amino)-5,7-dihydro-6H-pyrolo[3,4-d]pyrimidine-6-yl)(4-methoxytetrahydro-2H-pyran-4-yl)methanone.**

The compound of Example 3 was prepared by the reaction scheme below.

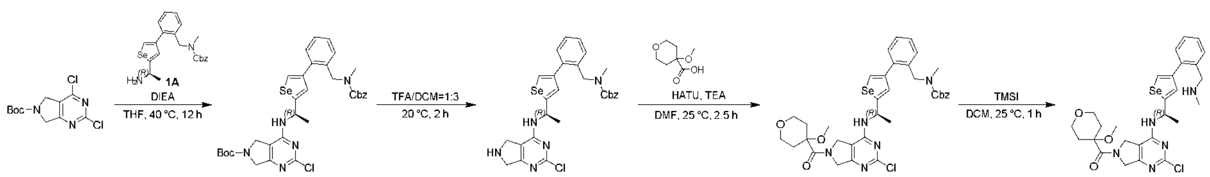


Step (1) Preparation of tert-butyl (R)-4-((1-(4-(2-((((benzyloxy)carbonyl)(methyl)amino)methyl)phenyl)selenophen-2-yl)ethyl)amino)-2-chloro-5,7-dihydro-6*H*-pyrolo[3,4-*d*]pyrimidine-6-carboxylate.

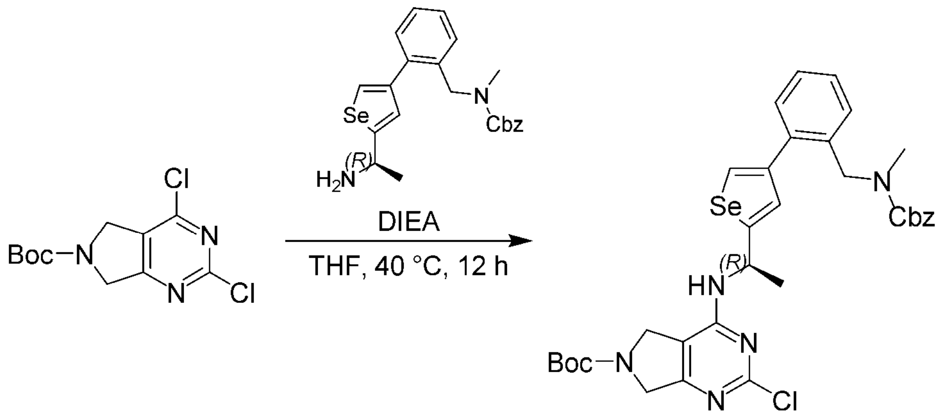


tert-Butyl 2,4-dichloro-5,7-dihydro-6H-pyrolo[3,4-d]pyrimidine-6-carboxylate (100 mg, 344 μmol, 1.00 eq) and benzyl (R)-(2-(5-(1-aminoethyl)selenophen-3-yl)benzyl)(methyl)carbamate (147 mg, 344 μmol, 1.00 eq) were dissolved in tetrahydrofuran (1.00 mL), and N,N-diisopropylethylamine (133 mg, 1.03 mmol, 180 μL, 3.00 eq) was added. The mixture was stirred at 40 °C for 12 h. The mixture was concentrated in vacuo to obtain a residue. The residue was purified by Prep-HPLC (column: YMC-Actus Triart C18 150 x 30 mm x 7 μm; mobile phase: [water (formic acid) - acetonitrile]; gradient: 80%-100% B for 10 min) and lyophilized to obtain tert-butyl (R)-4-((1-(4-(2-((((benzyloxy)carbonyl)(methyl) amino)methyl)phenyl)selenophen-2-yl)ethyl)amino)-2-chloro-5,7-dihydro-6H-pyrolo[3,4-d]pyrimidine-6-carboxylate (140 mg, 203 μmol, 59% yield, 99% purity) as a yellow solid.

$^1$H NMR (400 MHz, DMSO-$d_6$) $\delta$ = 8.30 (br d, $J$ = 5.6 Hz, 1H), 7.89 (br d, $J$ = 17.6 Hz, 1H), 7.43 - 7.24 (m, 8H), 7.23 - 7.10 (m, 2H), 5.60 - 5.47 (m, 1H), 5.14 - 4.95 (m, 2H), 4.48 (br s, 2H), 4.36 (br d, $J$ = 5.6 Hz, 4H), 2.76 (br s, 3H), 1.59 (br s, 3H), 1.45 (s, 9H).

Step (2) Preparation of benzyl (R)-(2-(5-(1-((2-chloro-6,7-dihydro-5H-pyrolo[3,4-*d*]pyrimidine-4-yl)amino)ethyl)selenophen-3-yl)benzyl)(methyl)carbamate

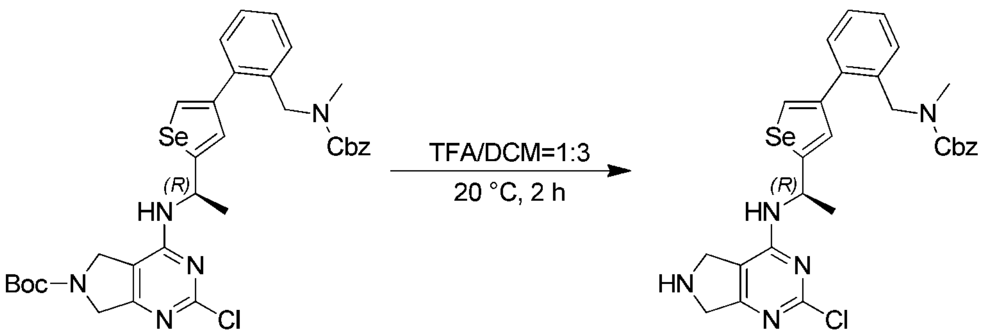


tert-Butyl (R)-4-((1-(4-(2-((((benzyloxy)carbonyl)(methyl)amino)methyl)phenyl)selenophen-2-yl)ethyl)amino)-2-chloro-5,7-dihydro-6H-pyrolo[3,4-d]pyrimidine-6-carboxylate (120 mg, 176 μmol, 1.00 eq) was dissolved in dichloromethane (1.20 mL) and trifluoroacetic acid (0.400 mL). The mixture was stirred at 20 °C for 2 h. The mixture was concentrated in vacuo to obtain the target compound, (R)-(2-(5-(1-((2-chloro-6,7-dihydro-5H-pyrolo[3,4-d]pyrimidine-4-yl)amino)ethyl)selenophen-3-yl)benzyl)(methyl) carbamate (102 mg) as a purple oil.

MS (ESI) m/z 582.2 $[M+H]^+$

Step (3) Preparation of benzyl (R)-(2-(5-(1-((2-chloro-6-(4-methoxytetrahydro-2H-pyran-4-carbonyl)-6,7-dihydro-5H-pyrolo[3,4-*d*]pyrimidine-4-yl)amino)ethyl)selenophen-3-yl)benzyl)(methyl)carbamate

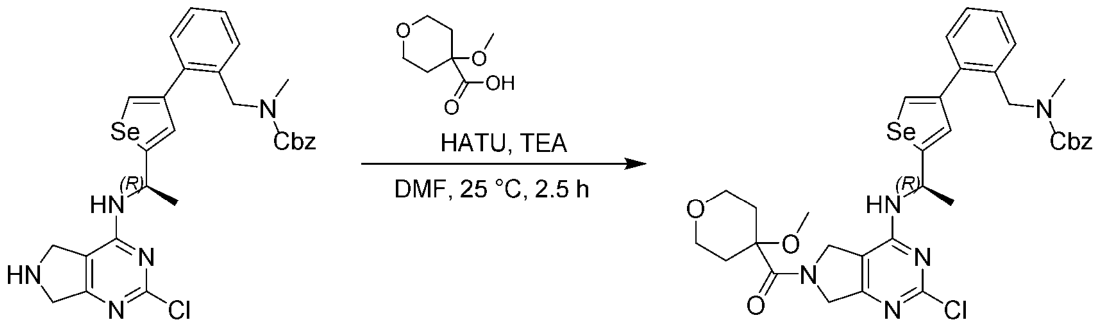


4-Methoxytetrahydro-2H-pyran-4-carboxylic acid (28.1 mg, 175 μmol, 1.00 eq) was dissolved in dimethylformamide (1.00 mL). 2-(3H-[1,2,3]-triazolo[4,5-b]pyridin-3-yl)-1,1,3,3-tetramethylisourea hexafluorophosphate (V) (100 mg, 263 μmol, 1.50 eq) and triethylamine (53.3 mg, 526 μmol, 73.3 μL, 3.00 eq) were added. The mixture was stirred at 25 °C for 0.5 h. Then, benzyl (R)-(2-(5-(1-((2-chloro-6,7-dihydro-5H-pyrolo[3,4-d]pyrimidine-4-yl)amino)ethyl)selenophen-3-yl)benzyl)(methyl)carbamate (102 mg, 175 μmol, 1.00 eq) and triethylamine (53.3 mg, 526 μmol, 73.3 μL, 3.00 eq) were added to the mixture. The mixture was stirred at 25 °C for 2 h. After filtering the mixture, the filtrate was purified by Prep-HPLC (Column: YMC-Actus Triart C18 150Г30 mm□7 □m; Mobile phase: [water (formic acid) - acetonitrile]; Gradient: 63%-93% B for 10 min) and lyophilized to obtain benzyl (R)-(2-(5-(1-((2-chloro-6-(4methoxytetrahydro-2H-pyran-4-carbonyl)-6,7-dihydro-5H-pyrolo[3,4-d]pyrimidine-4-yl)amino)ethyl)selenophen-3-yl)benzyl)(methyl)carbamate (100 mg, 136 μmol, 77% yield, 99% purity) as a white solid.

$^1$H NMR (400 MHz, DMSO-$d_6$) $\delta$ = 8.41 (br dd, $J$ = 3.2, 3.6 Hz, 1H), 8.00 - 7.81 (m, 1H), 7.40 - 7.22 (m, 8H), 7.21 - 7.09 (m, 2H), 5.61 - 5.47 (m, 1H), 5.14 - 4.94 (m, 2H), 4.78 (br s, 2H), 4.50 (br s, 4H), 3.75 - 3.51 (m, 4H), 3.17 - 3.11 (m, 3H), 2.76 (br s, 3H), 2.02 - 1.76 (m, 4H), 1.60 (br d, $J$ = 2.4 Hz, 3H).

Step (4) Preparation of (R)-(2-chloro-4-((1-(4-(2-((methylamino)methyl)phenyl)selenophen-2-yl)ethyl)amino)-5,7-dihydro-6*H*-pyrolo[3,4-*d*]pyrimidine-6-yl)(4-methoxytetrahydro-2*H*-pyran-4-yl)methanone

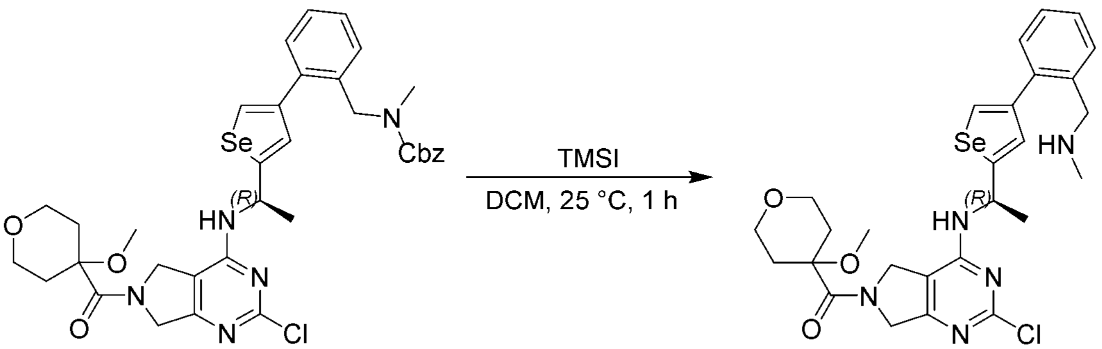


Benzyl(R)-(2-(5-(1-((2-chloro-6-(4-methoxytetrahydro-2H-pyran-4-carbonyl)-6,7-dihydro-5H-pyrolo[3,4-d]pyrimidine-4-yl)amino)ethyl)selenophen-3-yl)benzyl)(methyl)carbamate (90.0 mg, 124 μmol, 1.00 eq) was dissolved in dichloromethane (1.00 mL), and then iodotrimethylsilane (29.8 mg, 149 μmol, 20.3 μL, 1.20 eq) was added. The mixture was stirred at 25 °C for 1 h. The mixture was concentrated in vacuo to obtain the residue, which was purified by Prep-HPLC (Column: YMC-Actus Triart C18 150 x 30 mm x 7 μm; Mobile phase: [water(formic acid)-acetonitrile]; Gradient: 20%~50% B for 10 min) and lyophilized to obtain (R)-(2-chloro-4-((1-(4-(2-((methylamino)methyl)phenyl)selenophen-2-yl)ethyl)amino)-5,7-dihydro-6H-pyrolo[3,4-d]pyrimidine-6-yl)(4-methoxytetrahydro-2H-pyran-4-yl)methanone (25.46 mg, 32.9 μmol, 26.4% yield, 96.8% purity, formate) as a white solid.

$^1$H NMR (400 MHz, DMSO-$d_6$) $\delta$ = 8.48 - 8.37 (m, 1H), 8.21 (s, 1H), 8.05 - 7.98 (m, 1H), 7.52 - 7.47 (m, 1H), 7.41 - 7.28 (m, 4H), 5.64 - 5.51 (m, 1H), 4.80 (br s, 2H), 4.53 (br s, 2H), 3.71 (br s, 2H), 3.57 (br s, 4H), 3.17 - 3.13 (m, 3H), 2.32 (s, 3H), 1.97 - 1.81 (m, 4H), 1.66 - 1.58 (m, 3H).

**[Example 4]**

**Preparation of (R)-4-methyl-N-(1-(4-(2-((methylamino)methyl)phenyl)selenophen-2-yl)ethyl)-7-morpholinopyrido[3,4-d]pyridazine -1-amine**

The compound of Example 4 was prepared by the reaction scheme below.

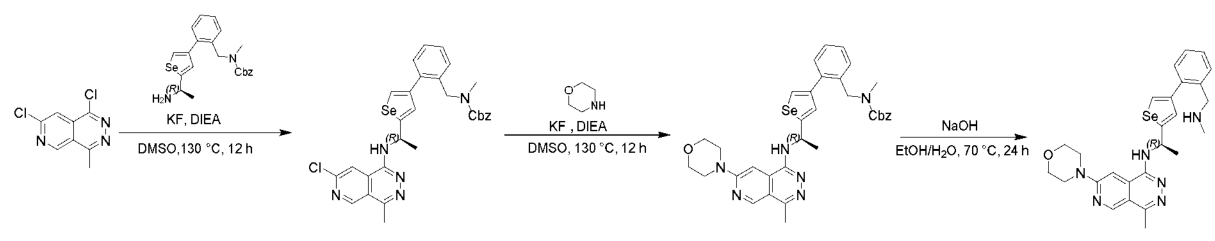


Step (1) Preparation of benzyl (R)-(2-(5-(1-((7-chloro-4-methylpyrido[3,4-*d*]pyridazine-1-yl)amino)ethyl)selenophen-3-yl)benzyl)(methyl) carbamate

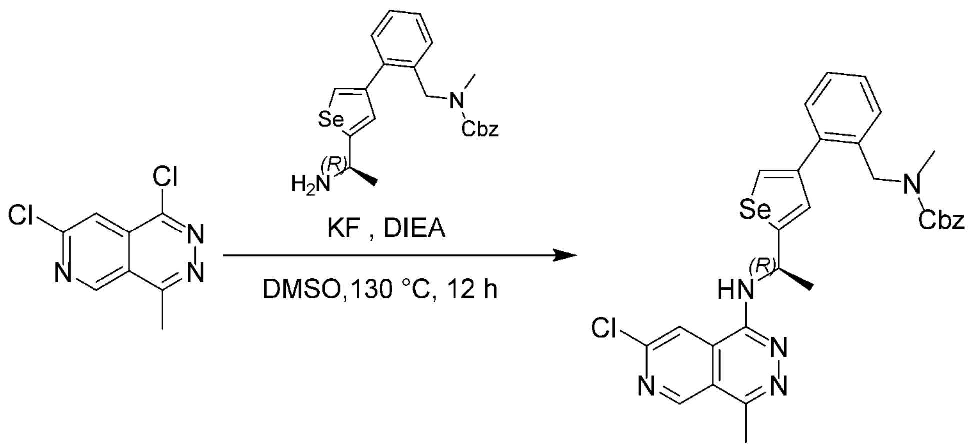


1,7-Dichloro-4-methylpyrido[3,4-d]pyridazine (0.400 g, 1.87 mmol, 1.00 eq) and benzyl (R)-(2-(5-(1-aminoethyl) selenophen-3-yl)benzyl)(methyl)carbamate (798 mg, 1.87 mmol, 1.00 eq) were dissolved in dimethyl sulfoxide (3.00 mL). Then, potassium fluoride (325 mg, 5.61 mmol, 3.00 eq) and N,N-diisopropylethylamine (724 mg, 5.61 mmol, 976 μL, 3.00 eq) were added. The mixture was stirred at 130°C for 12 h. The reaction mixture was quenched by adding water (10 mL), and then extracted with dichloromethane (2 × 15 mL). The organic layers were combined, washed with aqueous sodium chloride solution, and dried over anhydrous sodium sulfate. After filtration, the residue was concentrated in vacuo. The residue was purified by Prep-HPLC (column: Phenomenex luna C18 150 x 40 mm x 15 μm; mobile phase: [water(formic acid)-acetonitrile]; gradient: 38%-68% B for 15 min) and lyophilized to give benzyl (R)-(2-(5-(1-((7-chloro-4-methylpyrido [3,4-d]pyridazin-1-yl)amino)ethyl)selenophen-3-yl)benzyl)(methyl)carbamate (268 mg, 443 μmol, 23.7% yield) as a yellow solid.

$^1$H NMR (400 MHz, DMSO-$d_6$) $\delta$ = 9.32 (s, 1H), 8.55 (s, 1H), 7.40 - 7.26 (m, 10H), 7.21 - 7.15 (m, 2H), 5.84 (br t, $J$ = 6.8 Hz, 1H), 5.12 - 5.01 (m, 2H), 4.50 (br s, 2H), 2.79 (s, 6H), 1.74 (br s, 3H).

Step (2) Preparation of benzyl (R)-methyl(2-(5-(1-((4-methyl-7-morpholinopyrido[3,4-*d*]pyridazine-1-yl)amino)ethyl)selenophen-3-yl)benzyl)carbamate

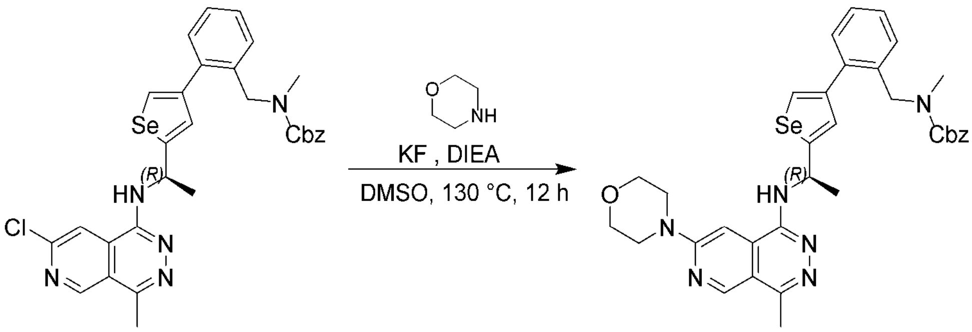


Morpholine (193 mg, 2.21 mmol, 195 μL, 5.00 eq) and benzyl (R)-(2-(5-(1-((7-chloro-4-methylpyrido[3,4-d]pyridazin-1-yl)amino)ethyl)selenophen-3-yl)benzyl)(methyl) carbamate (268 mg, 442 μmol, 1.00 eq) were dissolved in dimethyl sulfoxide (3.00 mL), and potassium fluoride (100 mg, 664 μmol, 24.5 μL, 1.50 eq) and diisopropylethylamine (171 mg, 1.33 mmol, 231 μL, 3.00 eq) were added. The mixture was stirred at 130 °C for 12 h, and then water (15 mL) was added. Subsequently, after extraction with dichloromethane (2 × 10 mL), the organic solvent was washed with saturated sodium chloride solution (2 × 10 mL). The organic solvent was dried over anhydrous sodium sulfate, filtered, and distilled under reduced pressure to obtain a residue. The residue was purified by Prep-HPLC (column: YMC-Actus Triart C18 150 x 30 mm x 7 μm; mobile phase: [water (formic acid) - acetonitrile; gradient: 35%~65% B for 10 min)) and lyophilized to obtain benzyl (R)-methyl(2-(5-(1-((4-methyl-7-morpholinopyrido[3,4-d]pyridazin-1-yl)amino)ethyl)selenophen-3-yl)benzyl)carbamate (70.0 mg, 106 μmol, 24.1% yield) as a yellow solid.

MS (ESI) m/z 657.2 $[M+H]^+$

Step (3) Preparation of (R)-4-methyl-N-(1-(4-(2-((methylamino)methyl)phenyl)selenophen-2-yl)ethyl)-7-morpholinopyrido[3,4-d] pyridazine-1-amine

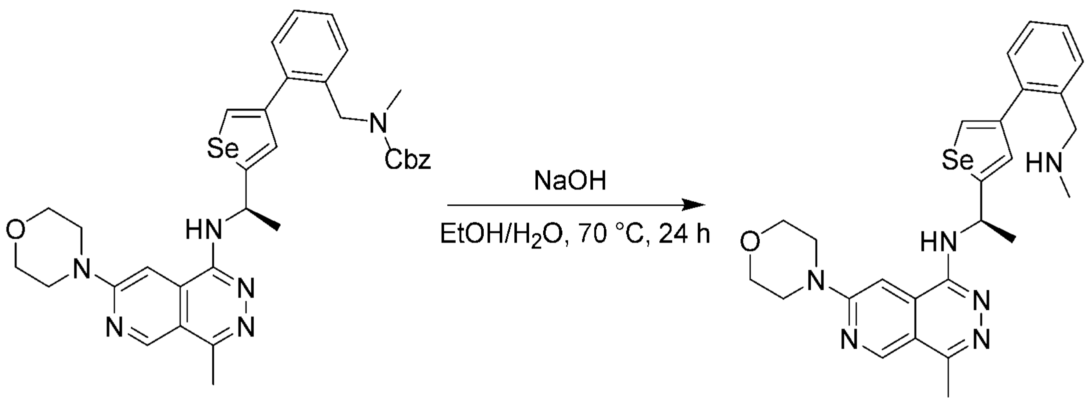


Benzyl (R)-methyl(2-(5-(1-((4-methyl-7-morpholinopyrido[3,4-d]pyridazin-1-yl)amino)ethyl)selenophen-3-yl)benzyl)carbamate (50.0 mg, 76.3 μmol, 1.00 eq) was dissolved in ethanol (2.00 mL)/water (0.200 mL), and sodium hydroxide (15.7 mg, 381.3 μmol, 45.7 μL, 5.00 eq) was added. The mixture was stirred at 70 °C for 24 h and then distilled under reduced pressure to obtain the residue. After purification of the entire product by Prep-HPLC (column: Phenomenex luna C18 150 x 25mm x 10 μm; mobile phase: [water(formic acid)-acetonitrile; gradient: 8%-38% B for 9 min), lyophilization was performed to obtain (R)-4-methyl-N-(1-(4-(2-((methylamino)methyl)phenyl)selenophen-2-yl)ethyl)-7-morpholinopyrido[3,4-d]pyridazin-1-amine (10.32 mg, 19.35 μmol, 25.38% yield, 97.8% purity, formate) as a yellow solid.

$^1H$ NMR (400 MHz, DMSO-$d_6$) $\delta$ = 9.04 (s, 1H), 8.23 (s, 1H), 7.94 (s, 1H), 7.54 (br d, $J$ = 6.4 Hz, 2H), 7.41 - 7.29 (m, 5H), 5.82 (br d, $J$ = 6.0 Hz, 1H), 3.81 - 3.75 (m, 6H), 3.68 (br d, $J$ = 4.8 Hz, 4H), 2.64 (s, 3H), 2.35 (s, 3H), 1.73 (d, $J$ = 6.8 Hz, 3H).

**[Example 5]**

**Preparation of *(R)-N-(1-(4-(2*-((dimethylamino)methyl)phenyl)selenophen-2-yl)ethyl)-6,7-dimethoxyl-2-methylquinazolin-4-amine**

The titled compound was prepared in a similar method of Example 1 according to the reaction scheme below.

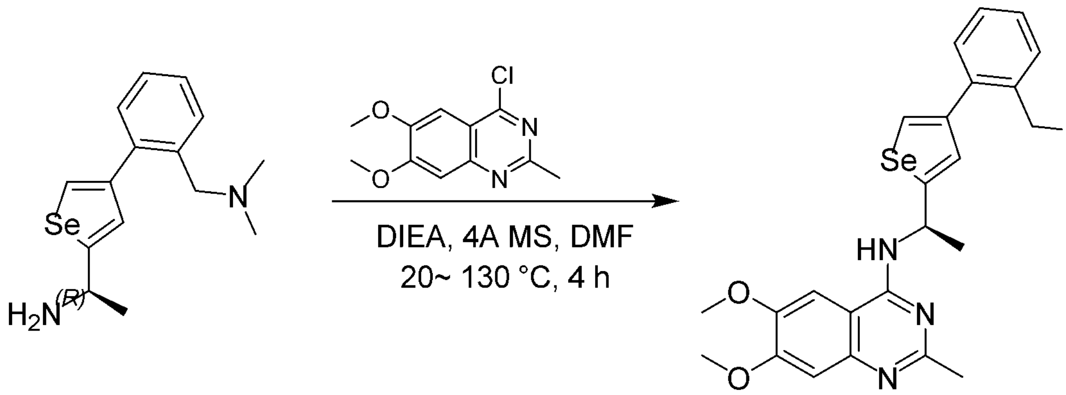


$^1$H NMR (400 MHz, DMSO-$d_6$) $\delta$ = 8.16 (d, $J$ = 8.0 Hz, 1H), 8.01 (d, $J$ = 1.2 Hz, 1H), 7.64 (s, 1H), 7.49 (t, $J$ = 1.2 Hz, 1H), 7.43 - 7.37 (m, 1H), 7.33 - 7.27 (m, 3H), 7.05 (s, 1H), 5.95 - 5.85 (m, 1H), 3.87 (d, $J$ = 2.0 Hz, 6H), 3.28 (d, $J$ = 5.2 Hz, 2H), 2.45 (s, 3H), 2.10 (s, 6H), 1.73 (d, $J$ = 6.8 Hz, 3H).

**[Example 6]**

**Preparation of (R)-6,7-dimethoxyl-2-methyl-N-(1-(4-(2-(pyrrolidine-1-ylmethyl)phenyl)selenophen-2-yl)ethyl) quinazolin-4-amine**

The titled compound was prepared in a similar method of Example 1 according to the reaction scheme below.

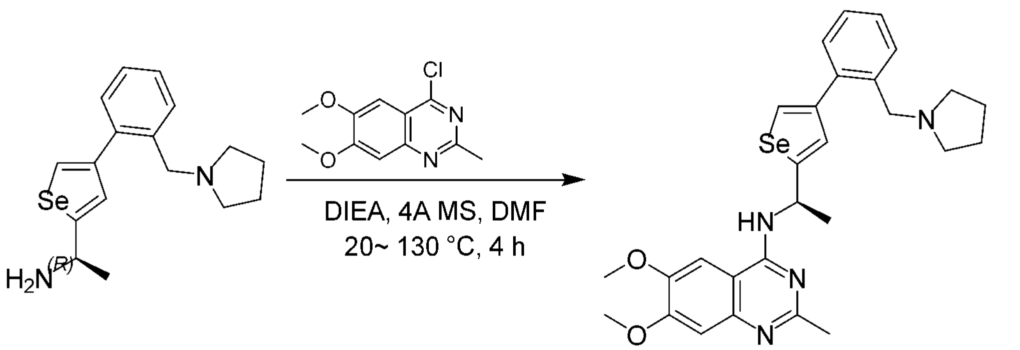


$^1$H NMR (400 MHz, DMSO-$d_6$) $\delta$ = 14.69 - 14.11 (m, 1H), 9.96 - 9.77 (m, 1H), 9.87 (br d, $J$ = 1.6 Hz, 1H), 8.06 (d, $J$ = 1.2 Hz, 1H), 7.94 (s, 1H), 7.67 (dd, $J$ = 3.6, 5.2 Hz, 1H), 7.53 - 7.45 (m, 2H), 7.43 (s, 1H), 7.40 - 7.32 (m, 1H), 7.21 (s, 1H), 6.07 (quin, $J$ = 6.8 Hz, 1H), 4.37 (br s, 2H), 3.97 (s, 3H), 3.93 (s, 3H), 3.34 (br d, $J$ = 4.8 Hz, 2H), 2.87 (br s, 2H), 2.65 (s, 3H), 1.80 (d, $J$ = 7.2 Hz, 3H), 1.78 (br d, $J$ = 3.6 Hz, 4H).

**[Example 7]**

**Preparation of (*R*)-6,7-dimethoxyl-2-methyl-*N*-(1-(4-(2-(((methyl-$d_3$)amino)methyl)phenyl)selenophen-2-yl) ethyl)quinazolin-4-amine**

The titled compound was prepared in a similar method of Example 1 according to the reaction scheme below.

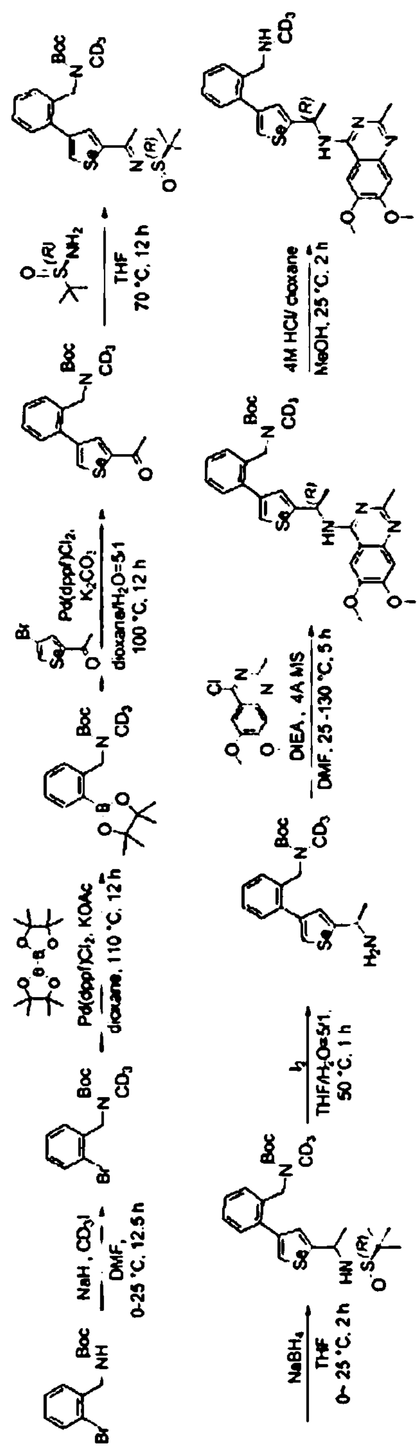


$^1H$ NMR (400 MHz, DMSO-$d_6$) $\delta$ = 8.21 (s, 2H), 8.17 (br d, $J$ = 8.0 Hz, 1H), 7.96 (s, 1H), 7.64 (s, 1H ), 7.52 (br d, $J$ = 7.2 Hz, 1H), 7.41 (s, 1H), 7.38 - 7.29 (m, 3H), 7.05 (s, 1H), 5.90 (quin, $J$ = 7.2 Hz, 1H), 3.87 (s, 6H), 3.76 (br s, 2H), 2.45 (s, 3H), 1.74 (d, $J$ = 6.8 Hz, 3H).

**[Example 8]**

**Preparation of *N*-((*R*)-1-(4-(2-((dimethylamino)methyl)phenyl)selenophen-2-yl)ethyl)-7-methoxy-2-methyl-6-(((*S*)-tetrahydrofuran-3-yl)oxy)quinazolin-4-amine**

The titled compound was prepared in a similar method of Example 2 according to the reaction scheme below.

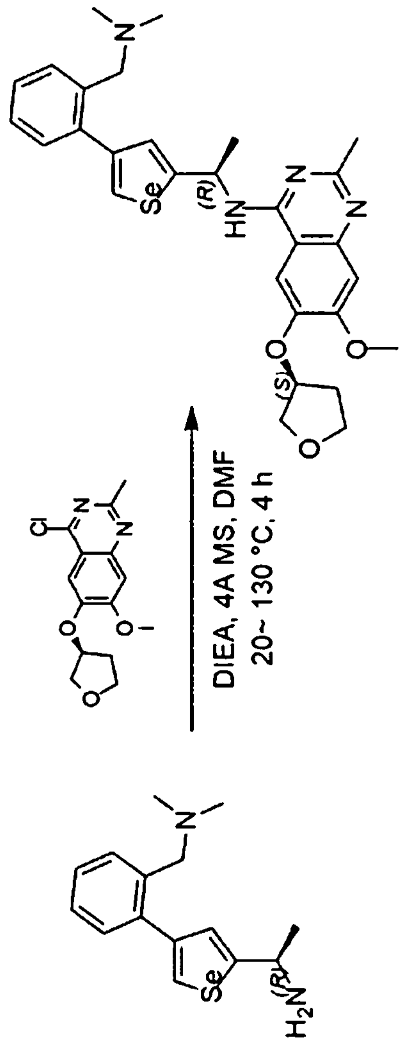


$^1$H NMR (400 MHz, DMSO-$d_6$) $\delta$ = 8.58 (d, $J$ = 8.0 Hz, 1H), 8.46 (s, 1H), 8.07 (s, 1H), 7.93 (s, 1H), 7.85 (br d, $J$ = 3.6 Hz, 1H), 7.76 - 7.73 (m, 3H), 7.52 (s, 1H), 6.39 - 6.34 (m, 1H), 5.58 - 5.55 (m, 1H), 4.42 - 4.39 (m, 1H), 4.32 (s, 3H), 4.31 - 4.23 (m, 3H), 3.73 (br d, $J$ = 4.4 Hz, 2H), 2.90 (s, 3H), 2.74 - 2.71 (m, 1H), 2.55 (s, 6H), 2.46 (br s, 1H), 2.18 (d, $J$ = 6.8 Hz, 3H).

**[Example 9]**

**Preparation of 7-methoxy-2-methyl-*N*-((*R*)-1-(4-(2-(pyrrolidine-1-ylmethyl)phenyl)selenophen-2-yl) ethyl)-6-(((*S*)-tetrahydrofuran-3-yl)oxy)quinazolin-4-amine**

The titled compound was prepared in a similar method of Example 2 according to the reaction scheme below.

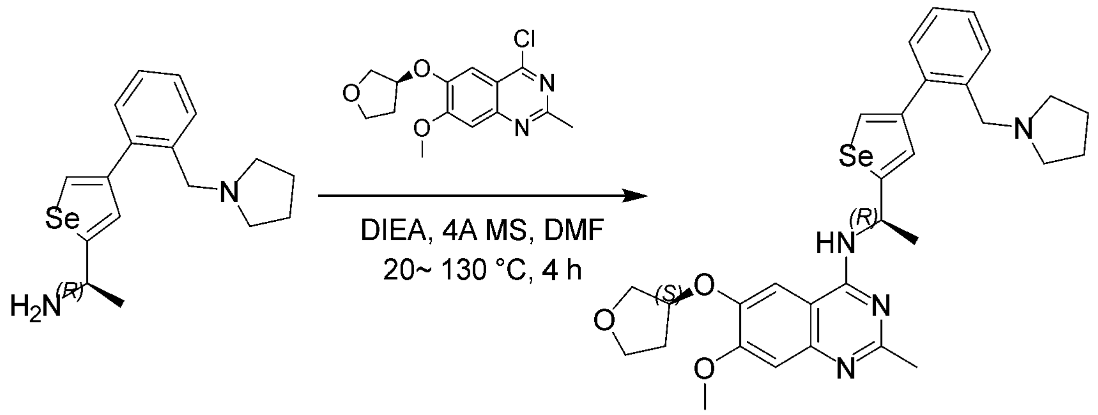


$^1H$ NMR (400 MHz, DMSO-$d_6$) $\delta$ = 8.17 - 8.11 (m, 1H), 8.02 (s, 1H), 7.62 (s, 1H), 7.55 (s, 1H), 7.43 - 7.40 (m, 1H), 7.32 - 7.27 (m, 3H), 7.08 (s, 1H), 5.92 (br t, *J* = 7.2 Hz, 1H), 5.13 (br d, *J* = 4.4 Hz, 1H), 3.96 (dd, *J* = 4.8, 10.0 Hz, 1H), 3.88 (s, 3H), 3.86 - 3.74 (m, 3H), 3.52 - 3.44 (m, 2H), 2.45 (s, 3H), 2.39 (br s, 4H), 2.31 - 2.24 (m, 1H), 2.01 (br dd, *J* = 6.4, 12.8 Hz, 1H), 1.73 (d, *J* = 6.8 Hz, 3H), 1.63 (br s, 4H).

**[Experimental Example 1]**

**Biochemical analysis of the inhibitory activity of SOS1 and KRAS G12C interaction**

In this experimental example, the efficacy of the compound according to the present invention in inhibiting the binding of KRAS and SOS1 was biochemically measured. A KRAS G12C working solution was prepared by adding biotin-linked KRAS G12C protein and europium-linked streptavidin to assay buffer solution (20 mM HEPES, 50 mM NaCl, 200 μg/mL fetal bovine serum (BSA), 0.01% Pluronic F127). Then, SOS1, in which a tag consisting of six histidine residues is covalently linked, and anti-histidine tag antibody D2 were added to the assay buffer to prepare an SOS1 working solution. After these two working solutions were added to the wells of a well plate, solutions diluted according to the experimental concentration of the test compound from the 10 mM dimethyl sulfoxide (DMSO) stock solution of the Example compound or positive control compound were added to the wells in 100 nL aliquots using an Echo automatic dispenser (Beckman Coulter, USA), or a DMSO blank was added to the wells. After incubation for 60 minutes at room temperature, the fluorescence signal intensity was measured at two emission wavelengths using a PerkinElmer Envision instrument (excitation wavelength: 320 nm, emission wavelength: 665/615 nm). As positive control compounds, BAY-293 [(S)-6,7-dimethoxy-2-methyl-N-[1-[4-[2-[(methylamino)methyl]phenyl]thiophene-2-yl]ethyl]quinazolin-4-amine] or MRTX0902 [(R)-2-methyl-3-(1-((4-methyl-7-morpholinopyrido[3,4-d]pyridazine-1-yl)amino)ethyl)benzonitrile], which are known SOS1 inhibitors, were used. The $IC_{50}$ values determined from the concentration curve of the inhibitory activity using the following relationship between the fluorescence measurement value y when the test compound concentration is x and the minimum value, maximum value, and half-inhibitory concentration (IC50) of the fluorescence measurement are shown in Table 1 below.

$$y = \text{Minimum fluorescence} + \frac{\text{Minimum fluorescence} - \text{Maximum fluorescence}}{1 + 10^{Hill\ \text{slope}\ \times (\log_{10} IC_{50} - x)}}$$

[Table 1]

| Compound | Enzyme activity $IC_{50}$ (nM) | Compound | Enzyme activity $IC_{50}$ (nM) |
|---|---|---|---|
| MRTX0902 | 32.96 | BAY-293 | 38.52 |
| Example 1 | 18.63 | Example 6 | 76.03 |
| Example 2 | 16.55 | Example 7 | 19.26 |
| Example 3 | 34.67 | Example 8 | 16.16 |
| Example 4 | 13.4 | Example 9 | 22.50 |

(continued)

| Compound | Enzyme activity $IC_{50}$ (nM) | Compound | Enzyme activity $IC_{50}$ (nM) |
|---|---|---|---|
| Example 5 | 27.7 | ** | ** |

**[Experimental Example 2]**

**Biochemical analysis of inhibitory activity against the interactions between SOS1 and KRAS (G12D, G12V, G13D, or wild-type proteins), and between SOS2 and KRAS G12C**

In this experimental example, the efficacy of the compound according to the present invention in inhibiting the binding of SOS1 to various types of mutant KRAS and wild-type KRAS and the inhibition activity of the interaction between KRAS G12C and SOS2 for testing selectivity of the compound for the SOS2 enzyme, were biochemically measured. SOS1 or SOS2 proteins covalently linked to a tag consisting of six histidine residues were added to assay buffer (10 mM HEPES, 150 mM NaCl, 0.05% fetal bovine serum (BSA), 10 mM EDTA, 0.0005% Triton X100, 1 mM DTT). A solution diluted at each experimental concentration of the test compound from a 10 mM dimethyl sulfoxide (DMSO) stock solution of the Example compound or positive control compound was added to the well using an Echo automatic dispenser (Beckman Coulter, USA), or a DMSO blank sample was added to the well. After 15 min of incubation at room temperature, a working solution containing a mixture of biotin-linked KRAS, XL665-linked streptavidin (Cisbio 610SAXLB), and a detection solution containing terbium (Tb)-labeled anti-histidine tag antibody (Cisbio 61HI2TLA) in assay buffer was added to the assay wells. After 45-60 min of incubation at room temperature, the fluorescence signal intensity was measured at two emission wavelengths using a PHERAstar instrument from BMG Labtech (excitation wavelength: 337 nm, emission wavelength: 665/620 nm). BAY-293 or MRTX0902, known SOS1 inhibitors, were used as positive control compounds. The $IC_{50}$ values determined from the concentration curve of inhibitory activity using the following relationship between the fluorescence measurement value y when the test compound concentration is x and the minimum and maximum values of measured fluorescence, and half-inhibitory concentrations (IC50) of the are shown in Table 2 below.

$$y = \text{Minimum fluorescence} + \frac{\text{Minimum fluorescence} - \text{Maximum fluorescence}}{1 + 10^{\text{Hill slope} \times (\log_{10} IC_{50} - x)}}$$

[Table 2]

| SOS subtype | KRAS mutant | Enzyme activity $IC_{50}$ (nM) | | |
|---|---|---|---|---|
| | | 38.52 | BAY-293 | Example 1 |
| SOS1 | G12C | 32.96 | 38.52 | 18.63 |
| | G12D | 16.24 | 11.78 | 8.4 |
| | G12V | 36.04 | 20.35 | 16.85 |
| | G13D | 20.98 | 15.96 | 12.51 |
| | WT | 18.65 | 15.75 | 9.56 |
| SOS2 | G12Cs | 1638 | NT | 24590 |

**[Experimental Example 3] Cell Growth Inhibition Assay**

In this experimental example, the cell growth inhibitory effect of compounds according to the present invention and positive control compounds in tumor cell lines was examined.

NCI-H358 (US ATCC catalog number CRL-5807), a cancer cell line harboring KRAS G12C mutation, was cultured in a cell culture medium containing RPMI1640 (US Gibco catalog number 22400-089) and 10% fetal bovine serum (US Excell Bio catalog number FSP500), and 1,000 cells were seeded per well in a 96-well 3D assay plate and cultured for 1 day at 37°C and 5% $CO_2$. From a 10-fold concentrated DMSO stock solution of the compound to be tested, a diluted amount of the test compound or DMSO blank was added to each well so that the final DMSO concentration was 0.25% in

the culture medium, and this was cultured in a cell culture incubator for 12 days. The cell culture medium containing the compound was replaced by 100 μL every 3 days. Luminescence analysis was performed using the 3D CellTiter-Glo Luminescent Cell Viability Assay Kit (Promega, USA) (Promega catalog number G9681). Luminescence was measured using a PerkinElmer 2014 EnVision plate reader. The percent cell growth inhibition rate was calculated from the measured relative luminescence unit (RLU) as follows.

$$Inhibition\ \ rate = 1 - \left( \frac{\text{RLU compound} - \text{RLU blank}}{\text{RLU control} - \text{RLU blank}} \right) \text{X } 100$$

From this cell growth inhibition rate, the half-maximal inhibitory concentration ($IC_{50}$), at which each compound inhibits cell growth by 50%, was calculated using the GraphPad Prism program and is summarized in Table 3 below.

[Table 3]

| Compound | Cell growth $IC_{50}$ (nM) | Compound | Cell growth $IC_{50}$ (nM) |
|---|---|---|---|
| MRTX0902 | 107.7 | BAY-293 | 135.6 |
| Example 1 | 20.7 | Example 5 | 50.0 |
| Example 2 | 58.7 | Example 6 | 87.5 |
| Example 3 | 123.5 | Example 7 | 25.6 |
| Example 4 | 51.7 | ** | ** |

**[Experimental Example 4]**

**Test for evaluating the combination effect of targeted anticancer agent- Cell Viability**

In this experimental example, we examined whether the compound according to the present invention has a synergistic effect on cancer cell death when used in combination with a target anticancer agent related to another RAS signal transduction pathway.

NCI-H358 cell line (ATCC catalog no. CRL-5807, USA) or EGFR exon 19 deletion cancer cell line PC-9 (Riken, Japan catalog no. RCB4455) were seeded in a well plate (500 cells per well for H358 and 50 cells per well for PC-9) in RPMI1640 (Gibco, USA catalog no. 22400-089) medium containing 10% fetal bovine serum (Excel Bio, USA catalog no. FSP500) and cultured for one day at 37°C under 5% CO2 conditions. Subsequently, the target anticancer agent and the compound of the present invention were added in an amount of 150 μL according to the final experimental concentration so that the DMSO concentration in the culture medium was 0.25%. The targeted anticancer drugs used were sotorasib, a Ras inhibitor (Selleck, USA, catalog number S8830), trametinib, a MEK inhibitor (MedChemExpress, USA, catalog number HY10999), and osimertinib, an EGFR inhibitor (MedChemExpress, USA, catalog number HY15772). Cell survival was observed at six concentrations (10, 1, 0.1, 0.01, 0.001, 0.0001 μM) for each of these compounds. Cell lines with added compounds were cultured in a cell culture medium for 7 days, and the cell culture medium containing the compounds was replaced on the 4th day of culture. Cell survival analysis was performed by luminescence analysis using the Promega CellTiter-Glo Luminescent Cell Viability Assay Kit (Promega, catalog number G7573), and luminescence was measured using a PerkinElmer 2014 EnVision plate reader. The inhibition rate was calculated from the measured relative luminescence value in the same manner as in Experimental Example 3, and the combination index (CI) between the compound of the present invention and the target anticancer agent was calculated using the following equation.

$$CI(x) = \frac{D_{\text{Targeted anticancer agent}}^{\text{Combined}}}{Dx_{\text{Targeted anticancer agent}}^{\text{Single}}} + \frac{D_{\text{Compound}}^{\text{Combined}}}{Dx_{\text{Compound}}^{\text{Single}}}$$

In the above equation, CI(x) is a combination index when the cell growth inhibition rate is x as measured in the combination experiment in which the concentrations of the targeted anticancer agent and the compound of the present invention are represented by $D_{\text{Targeted anticancer agent}}^{\text{Combined}}$ Targeted anticancer agent and $D_{\text{Compound}}^{\text{Combined}}$

respectively. $Dx_{\text{Targeted anticancer agent}}^{\text{Single}}$ is the concentration of the targeted anticancer agent required to achieve the same growth inhibition rate of x when the targeted anticancer agent is used alone, and $Dx_{\text{Compound}}^{\text{Single}}$ is the concentration of the compound required to achieve the same growth inhibition rate of x when the compound of the present invention is used alone.

In the combination test with a KRAS signaling blocking targeted anticancer drug targeting H358 cells, shown in Fig. 1, a strong synergistic effect was observed in all quantum concentration combinations between Example 1 compound in the range of 0.0001 to 0.1 μM and sotorasib in the range of 0.001 to 1 μM, inhibiting cell growth. On the other hand, trametinib showed a strong synergistic effect in all quantum concentration combinations between Example 1 compound in the range of 0.0001 to 0.1 μM and trametinib in the range of 0.001 to 0.1 μM, inhibiting cell growth. In a combination test with an EGFR signaling blocking targeted anticancer drug targeting PC-9 cells, shown in Fig. 2, a strong synergistic effect was observed in all quantum combinations between osimertinib in the range of 0.01 to 0.1 μM and Example compound in the range of 0.0001 to 10 μM.

From these results, it was expected that the compound of the present invention could block the binding of SOS1 and RAS, and would have a synergistic anticancer effect when used in combination with KRAS signaling pathway or EGFR signaling pathway inhibition therapy.

**[Experimental Example 5]**

**Reactive oxygen species (ROS) production assay**

In this experimental example, it was examined that the compound according to the present invention has the effect of increasing reactive oxygen in tumor cells.

MIA-PaCa-2 (Korea Cell Line Bank KCLB No. 21420), a cancer cell line harboring KRAS G12C mutation, was cultured in a cell culture medium containing RPMI1640 (Gibco, USA, catalog no. 11875-119), 10% fetal bovine serum (HyClone, USA, catalog no. SH30919.03), and 1 × diluted HyClone Antibiotic Antimycotic solution (HyClone, USA, catalog no. SV30079.01). 3 × 105 cells were seeded per well in a 6-well plate and cultured for 1 day at 37°C under 5% $CO_2$ conditions. Test compounds or DMSO blanks were added to each well so that the final concentration of the test compound was 10 μM and the final DMSO concentration was 0.2% in the culture medium, and the cells were cultured in a cell culture incubator for 24 hours. After washing once with phosphate buffered saline (PBS, Gibco, USA, catalog number 10010-049), the wells were treated with 20 μM 2',7'-dichlorodihydrofluorescein diacetate (Sigma-Aldrich, USA, catalog number D6883) for 30 minutes. The cells were then collected in a microtube and analyzed for fluorescence using a BD LSRFortessa™ X-20 Cell Analyzer (BD Biosciences, USA) (excitation wavelength: 488 nm, emission wavelength: 535 nm). The fluorescence intensity due to the increase in reactive oxygen species was measured for 10,000 cells. Among the cells treated with 500 μM hydrogen peroxide, which was the positive control, the section in which the fluorescence intensity increased compared to the cells treated with the blank sample was found, and the average fluorescence intensity of the cells within this section was calculated. Then, the average of the measured values of the increased fluorescence intensity compared to the blank sample within the same section of the cells treated with the test compound was converted into a percentage of the average of the positive control group.

In the graph of reactive oxygen species production rates shown in Figure 3, the compound of Example 1 significantly increased ROS production compared to the blank sample, while the control drug, MRTX0902, did not increase ROS production compared to the blank sample.

These results suggest that the compounds of the present invention increase intracytoplasmic ROS, which enhances their cell growth inhibitory effects.

Although the present invention has been described through limited embodiments as described above, the technical idea and scope of the present invention are not limited to these embodiments, and various modifications or variations of the invention described in the claims below, which are obvious to those skilled in the art, are also included in the scope of the present invention within the equivalent scope of the invention described in the claims.

**Claims**

**1.** A compound of formula 1 or a tautomer thereof and pharmaceutically acceptable salts thereof:

[Chemical Formula 1]

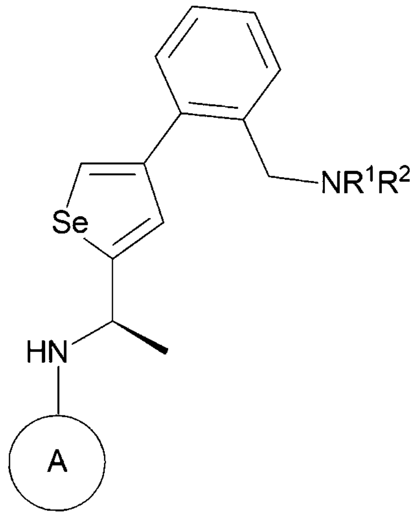

in the Chemical Formula 1, $R^1$ and $R^2$ are functional groups independently selected from the group consisting of H, $C_{1\sim4}$ alkyl, and $C_{1\sim4}$ alkyl substituted with deuterium, or $R^1$ and $R^2$ form a 4 to 6 membered heterocyclic alkyl ring together with nitrogen atom to which $R^1$ and $R^2$ are bonded, except that both of $R^1$ and $R^2$ are H,

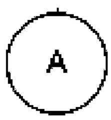

is

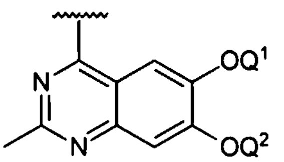 , 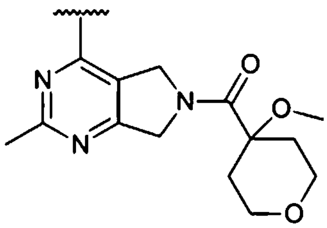 or 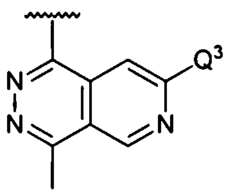

wherein $Q^1$ and $Q^2$ are functional groups independently selected from $C_{1\sim4}$ alkyl and 4 to 8 membered monocyclic heterocyclyl, $Q^3$ is 4 to 8 membered monocyclic heterocyclyl, and symbol "∿∿∿∿∿∿" indicates the position that Ⓐ is connected to remaining part of the compound.

2. The compound according to claim 1, wherein the 4 to 6 membered heterocyclic alkyl ring is azetidinyl or pyrrolidinyl.

3. The compound according to claim 1, **characterized in that** both $Q^1$ and $Q^2$ are methyl, or one of $Q^1$ and $Q^2$ is methyl and the other is tetrahydrofuranyl.

4. A compound selected from the group consisting of the following compounds, a stereoisomer, a tautomer, a solvate thereof or a pharmaceutically acceptable salt thereof:

(*R*)-6,7-dimethoxyl-2-methyl-*N*-(1-(4-(2-((methylamino)methyl)phenyl)selenophen-2-yl)ethyl)quinazolin-4-amine;
7-methoxy-2-methyl-*N*-((*R*)-1-(4-(2-((methylamino)methyl)phenyl)selenophen-2-yl)ethyl)-6-(((*S*)-tetrahydrofuran-3-yl)oxy)quinazolin-4-amine;
(*R*)-(2-chloro-4-((1-(4-(2-((methylamino)methyl)phenyl)selenophen-2-yl)ethyl)amino)-5,7-dihydro-6*H*-pyrolo[3,4-*d*]pyrimidine-6-yl)(4-methoxytetrahydro-2*H*-pyran-4-yl)methanone;

(*R*)-4-methyl-N-(1-(4-(2-((methylamino)methyl)phenyl)selenophen-2-yl)ethyl)-7-morpholinopyrido[3,4-d]pyridazine-1-amine;
(*R*)-*N*-(1-(4-(2-((dimethylamino)methyl)phenyl)selenophen-2-yl)ethyl)-6,7-dimethoxyl-2-methylquinazolin-4-amine;
(*R*)-6,7-dimethoxyl-2-methyl-*N*-(1-(4-(2-(pyrrolidine-1-ylmethyl)phenyl)selenophen-2-yl)ethyl)quinazolin-4-amine;
(*R*)-6,7-dimethoxyl-2-methyl-*N*-(1-(4-(2-(((methyl-$d_3$)amino)methyl)phenyl)selenophen-2-yl)ethyl)quinazolin-4-amine;
*N*-((*R*)-1-(4-(2-((dimethylamino)methyl)phenyl)selenophen-2-yl)ethyl)-7-methoxy-2-methyl-6-(((*S*)-tetrahydrofuran-3-yl)oxy)quinazolin-4-amine, and
7-methoxy-2-methyl-*N*-((*R*)-1-(4-(2-(pyrrolidine-1-ylmethyl)phenyl)selenophen-2-yl)ethyl)-6-(((*S*)-tetrahydrofuran-3-yl)oxy)quinazolin-4-amine.

**5.** A pharmaceutical composition for treatment of cancer, comprising a therapeutically effective amount of a compound according to claim 1, and a pharmaceutically acceptable excipient.

【FIG. 1】

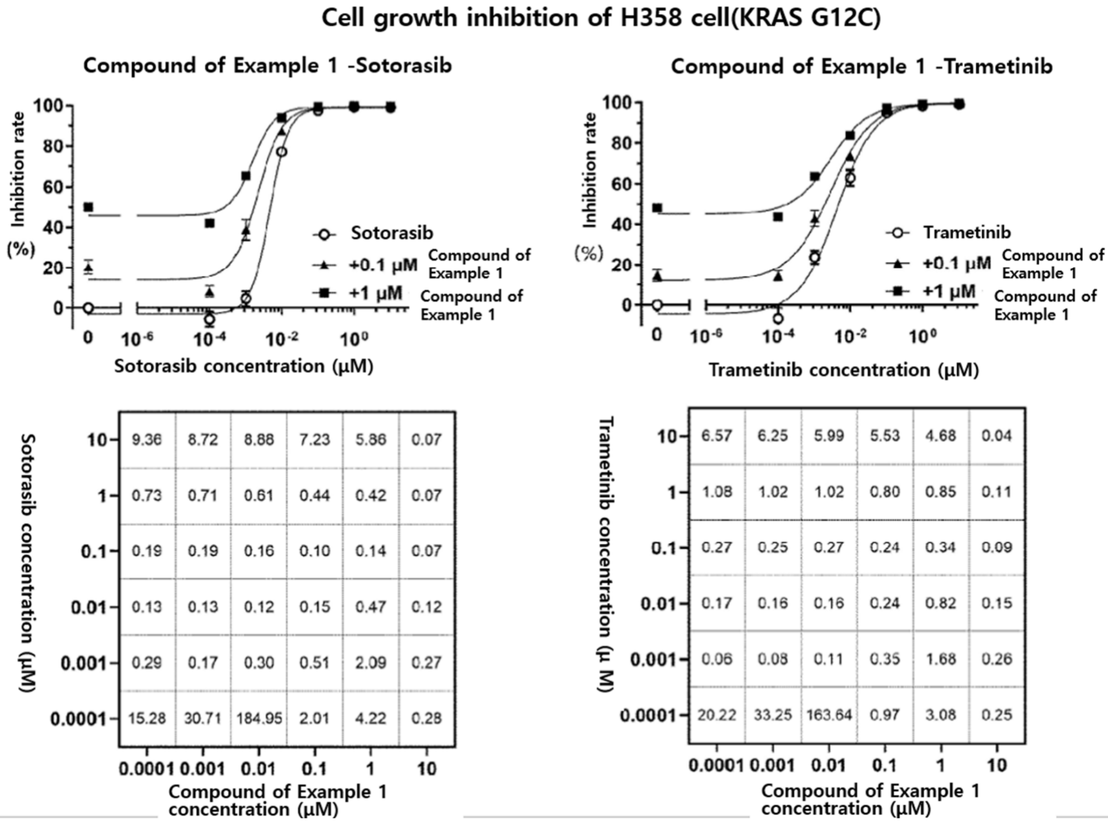
Cell growth inhibition of H358 cell(KRAS G12C)
Compound of Example 1 -Sotorasib
Inhibition rate (%)
100
80
60
40
20
0
Sotorasib
+0.1 μM Compound of Example 1
+1 μM Compound of Example 1
0
$10^{-6}$
$10^{-4}$
$10^{-2}$
$10^{0}$
Sotorasib concentration (μM)
Compound of Example 1 -Trametinib
Inhibition rate (%)
Trametinib
+0.1 μM Compound of Example 1
+1 μM Compound of Example 1
Trametinib concentration (μM)
Sotorasib concentration (μM)
10: 9.36 8.72 8.88 7.23 5.86 0.07
1: 0.73 0.71 0.61 0.44 0.42 0.07
0.1: 0.19 0.19 0.16 0.10 0.14 0.07
0.01: 0.13 0.13 0.12 0.15 0.47 0.12
0.001: 0.29 0.17 0.30 0.51 2.09 0.27
0.0001: 15.28 30.71 184.95 2.01 4.22 0.28
0.0001 0.001 0.01 0.1 1 10
Compound of Example 1 concentration (μM)
Trametinib concentration (μ M)
10: 6.57 6.25 5.99 5.53 4.68 0.04
1: 1.08 1.02 1.02 0.80 0.85 0.11
0.1: 0.27 0.25 0.27 0.24 0.34 0.09
0.01: 0.17 0.16 0.16 0.24 0.82 0.15
0.001: 0.06 0.08 0.11 0.35 1.68 0.26
0.0001: 20.22 33.25 163.64 0.97 3.08 0.25
0.0001 0.001 0.01 0.1 1 10
Compound of Example 1 concentration (μM)

【FIG. 2】

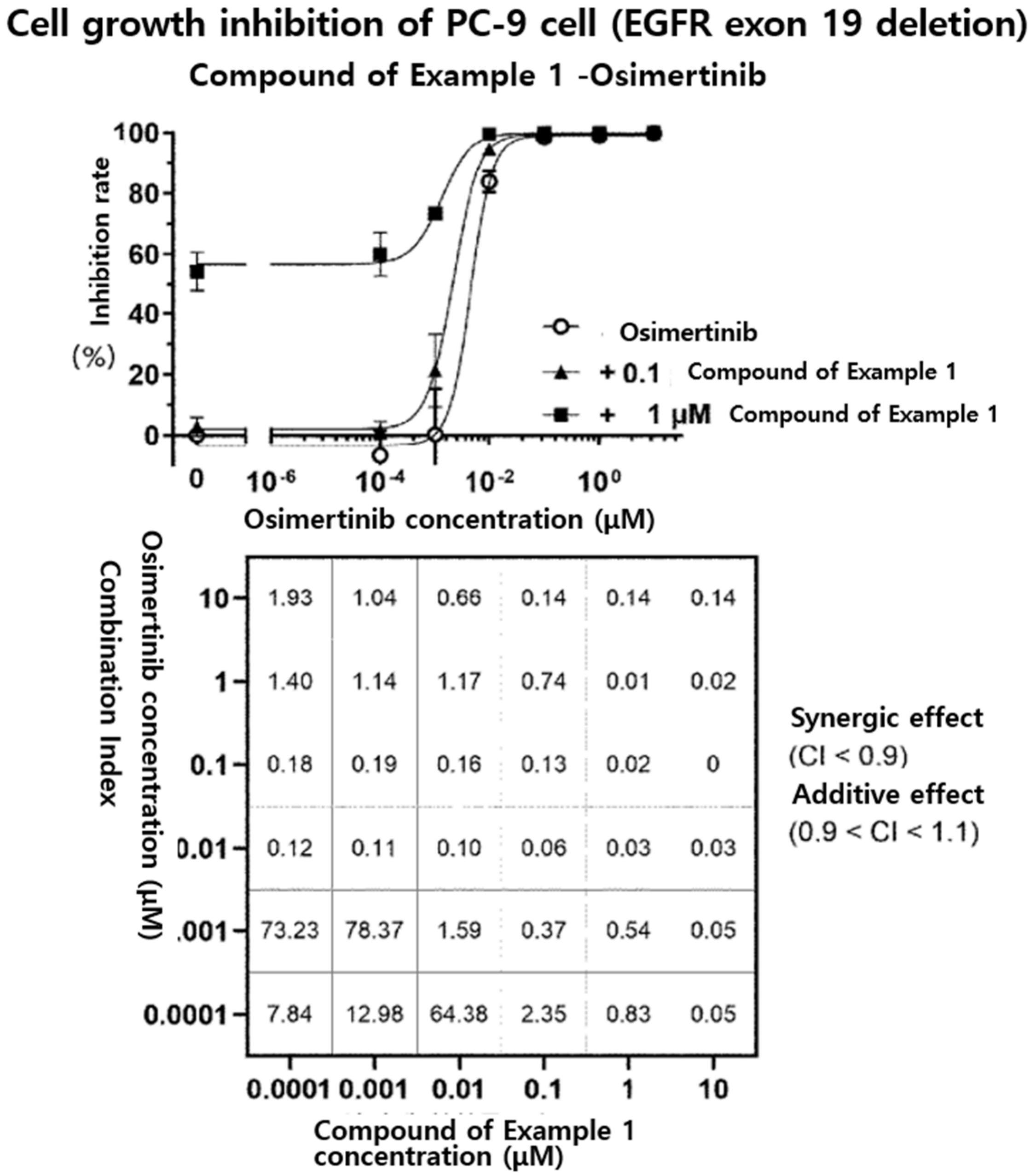
Cell growth inhibition of PC-9 cell (EGFR exon 19 deletion)
Compound of Example 1 -Osimertinib
Inhibition rate (%)
100
80
60
40
20
0
0
10-6
10-4
10-2
100
Osimertinib concentration (µM)
Osimertinib
+ 0.1 Compound of Example 1
+ 1 µM Compound of Example 1
Combination Index
Osimertinib concentration (µM)
10
1
0.1
0.01
.001
0.0001
1.93 1.04 0.66 0.14 0.14 0.14
1.40 1.14 1.17 0.74 0.01 0.02
0.18 0.19 0.16 0.13 0.02 0
0.12 0.11 0.10 0.06 0.03 0.03
73.23 78.37 1.59 0.37 0.54 0.05
7.84 12.98 64.38 2.35 0.83 0.05
0.0001 0.001 0.01 0.1 1 10
Compound of Example 1 concentration (µM)
Synergic effect
(CI < 0.9)
Additive effect
(0.9 < CI < 1.1)

【FIG. 3】

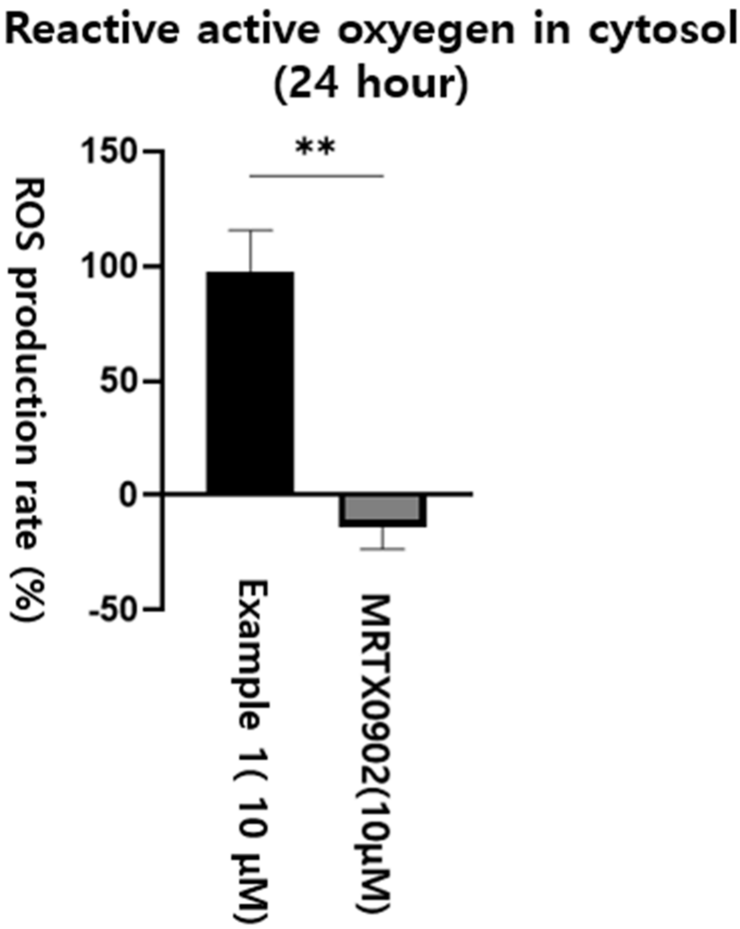
Reactive active oxyegen in cytosol
(24 hour)
ROS production rate (%)
150
100
50
0
-50
**
Example 1( 10 µM)
MRTX0902(10µM)

**INTERNATIONAL SEARCH REPORT**

International application No.
**PCT/KR2024/009146**

**A. CLASSIFICATION OF SUBJECT MATTER**

**C07D 421/12**(2006.01)i; **A61K 31/517**(2006.01)i; **A61K 31/519**(2006.01)i; **A61P 35/00**(2006.01)i; **C07D 421/14**(2006.01)i; **C07D 487/04**(2006.01)i; **A61K 31/095**(2006.01)i; **A61K 31/5377**(2006.01)i; **A61K 31/675**(2006.01)i; **A61K 31/695**(2006.01)i

According to International Patent Classification (IPC) or to both national classification and IPC

**B. FIELDS SEARCHED**

Minimum documentation searched (classification system followed by classification symbols)

C07D 421/12(2006.01); A61K 31/506(2006.01); A61K 38/20(2006.01); C07C 245/12(2006.01); C07D 345/00(2006.01); C07D 517/00(2006.01); C12Q 1/6886(2018.01); G01N 33/574(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: 셀레노펜(selenophene), 페닐(phenyl), 퀴나졸린(quinazoline), 파이롤로피리미딘(pyrrolopyrimidine), 피리도피리다진(pyridopyridazine), 암(cancer)

**C. DOCUMENTS CONSIDERED TO BE RELEVANT**

| Category* | Citation of document, with indication, where appropriate, of the relevant passages | Relevant to claim No. |
|---|---|---|
| A | KR 10-2013-0115302 A (KASINA LAILA INNOVA PHARMACEUTICALS PRIVATE LIMITED) 21 October 2013 (2013-10-21)<br>See claims 1, 25 and 33; and page 38. | 1-5 |
| A | US 2010-0272678 A1 (GOKARAJU, G. R. et al.) 28 October 2010 (2010-10-28)<br>See claims 1 and 23; and example 1. | 1-5 |
| A | CN 109554468 A (PEKING UNIVERSITY) 02 April 2019 (2019-04-02)<br>See abstract; and page 15. | 1-5 |
| A | WO 2023-005267 A1 (CHINA PHARMACEUTICAL UNIVERSITY) 02 February 2023 (2023-02-02)<br>See claims 1-10. | 1-5 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 October 2024** | **07 October 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/009146**

**C. DOCUMENTS CONSIDERED TO BE RELEVANT**

| Category* | Citation of document, with indication, where appropriate, of the relevant passages | Relevant to claim No. |
|---|---|---|
| A | ZHANG. S. et al. Selenophenes: Introducing a new element into the core of non-steroidal estrogen receptor ligands. Chemmedchem. 2017, vol. 12, pp. 235-249. See abstract; figures 1 and 2; and scheme 1. | 1-5 |

Form PCT/ISA/210 (second sheet) (July 2022)

**INTERNATIONAL SEARCH REPORT**
**Information on patent family members**

International application No.
**PCT/KR2024/009146**

| Patent document cited in search report | Publication date (day/month/year) | Patent family member(s) | Publication date (day/month/year) |
|---|---|---|---|
| KR 10-2013-0115302 A | 21 October 2013 | AU 2011-330730 A1 | 04 July 2013 |
| | | AU 2011-330730 B2 | 18 February 2016 |
| | | AU 2011-340063 A1 | 04 July 2013 |
| | | AU 2011-340063 B2 | 24 March 2016 |
| | | BR 112013011689 A2 | 17 October 2017 |
| | | CA 2818389 A1 | 24 May 2012 |
| | | CA 2820851 A1 | 14 June 2012 |
| | | CN 103221048 A | 24 July 2013 |
| | | CN 103313599 A | 18 September 2013 |
| | | CN 103313599 B | 23 March 2016 |
| | | EP 2640392 A2 | 25 September 2013 |
| | | EP 2640392 B1 | 07 January 2015 |
| | | EP 2648509 A2 | 16 October 2013 |
| | | EP 2648509 B1 | 29 April 2015 |
| | | HK 1185513 A1 | 21 February 2014 |
| | | HK 1185536 A1 | 21 February 2014 |
| | | IL 226783 A | 29 August 2013 |
| | | JP 2013-542982 A | 28 November 2013 |
| | | JP 2013-544881 A | 19 December 2013 |
| | | JP 5898687 B2 | 06 April 2016 |
| | | JP 5898689 B2 | 06 April 2016 |
| | | KR 10-1817221 B1 | 10 January 2018 |
| | | KR 10-1850729 B1 | 20 April 2018 |
| | | KR 10-2013-0126653 A | 20 November 2013 |
| | | RU 2013127577 A | 27 December 2014 |
| | | RU 2013131268 A | 20 January 2015 |
| | | RU 2566293 C2 | 20 October 2015 |
| | | RU 2597609 C2 | 10 September 2016 |
| | | US 2013-0266563 A1 | 10 October 2013 |
| | | US 2013-0287767 A1 | 31 October 2013 |
| | | US 8815879 B2 | 26 August 2014 |
| | | US 9090633 B2 | 28 July 2015 |
| | | WO 2012-066578 A2 | 24 May 2012 |
| | | WO 2012-066578 A3 | 19 July 2012 |
| | | WO 2012-077135 A2 | 14 June 2012 |
| | | WO 2012-077135 A3 | 04 October 2012 |
| US 2010-0272678 A1 | 28 October 2010 | AU 2010-243213 A1 | 27 October 2011 |
| | | AU 2010-243213 B2 | 11 September 2014 |
| | | BR PI1006651 A2 | 25 August 2015 |
| | | CA 2759519 A1 | 04 November 2010 |
| | | CN 102438449 A | 02 May 2012 |
| | | CN 102438449 B | 18 June 2014 |
| | | EP 2424351 A1 | 07 March 2012 |
| | | EP 2424351 B1 | 21 October 2015 |
| | | HK 1166591 A1 | 02 November 2012 |
| | | IL 215789 A | 31 January 2012 |
| | | JP 2012-525329 A | 22 October 2012 |
| | | JP 5571168 B2 | 13 August 2014 |
| | | KR 10-2012-0006554 A | 18 January 2012 |
| | | NZ 595602 A | 31 May 2013 |

Form PCT/ISA/210 (patent family annex) (July 2022)

**INTERNATIONAL SEARCH REPORT**
**Information on patent family members**

International application No.
**PCT/KR2024/009146**

| Patent document cited in search report | Publication date (day/month/year) | Patent family member(s) | Publication date (day/month/year) |
|---|---|---|---|
| | | US 8143237 B2 | 27 March 2012 |
| | | WO 2010-125575 A1 | 04 November 2010 |
| CN 109554468 A | 02 April 2019 | CN 109554468 B | 12 October 2021 |
| WO 2023-005267 A1 | 02 February 2023 | AU 2024-317677 A1 | 14 March 2024 |
| | | CA 3227135 A1 | 02 February 2023 |
| | | CN 113429387 A | 24 September 2021 |
| | | CN 113429387 B | 28 October 2022 |
| | | EP 4378935 A1 | 05 June 2024 |
| | | JP 2024-525976 A | 12 July 2024 |
| | | KR 10-2024-0041980 A | 01 April 2024 |
| | | US 2024-0051934 A1 | 15 February 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5846514 A
- US 6334997 B


### Non-patent literature cited in the description


- **JENG et al.** *Nat. Commun.*, 2012, vol. 3, 1168
- **JENG et al.** *Nat. Commun.*, 2012, vol. 3 (1), 168
- **BERGE et al.** *J. Pharmaceutical Sciences*, 1977, vol. 66, 1-19
- **LOUIS F. FIESER** ; **MARY FIESER**. Reagents for Organic Synthesis. Wiley, 1967
- Beilsteins Handbuch der organischen Chemie. Springer-Verlag
- Recent Advances in the Synthesis and Applications of Radiolabeled Compounds for Drug Discovery and Development. Curr., Pharm. Des. 2000, vol. 6, 10